# EUROPÄISCHE PATENTANMELDUNG

(11) **EP 2 452 706 A2**
(43) Veröffentlichungstag der Anmeldung: **16.05.2012**
(21) Anmeldenummer: 11075240.9
(22) Anmeldetag: 26.10.2011
(51) Int. Cl.: A61M 3/02

(54) **Darmrohr- Anordnung zur Applikation einer Flüssigkeit**

(30) Priorität: 26.10.2010 DE 102010060168; 27.04.2011 DE 102011100459
(71) Anmelder: Schlenzka, Reinhard, 10625 Berlin (DE)
(72) Erfinder: Schlenzka, Reinhard, 10625 Berlin (DE)

(57) **Zusammenfassung**

Es wird eine Darmrohr-Anordnung zur Applikation einer Flüssigkeit in den Dickdarm eines Lebewesens offenbart, wobei die Darmrohr-Anordnung ein Griffteil (109) und einen mit dem Griffteil (109) verbundenen Spülkörper (108) umfasst, wobei der Spülkörper (108) einen Fluidkanal (40) umfasst. Der Spülkörper (108) umfasst mindestens zwei in den Dickdarm eines Lebewesens einführbare Segmente (201, 202, 203). Jedes Segment (201, 202, 203) umfasst eine Mittelachse (118), eine Grundfläche (117, 127, 137), eine Deckfläche (116, 126, 136), einen ersten Bereich (111) und einen zweiten Bereich (112), wobei der erste Bereich (111) zwischen der Grundfläche (117, 127, 137) und dem zweiten Bereich (112) angeordnet ist und der zweite Bereich (112) zwischen dem ersten Bereich (111) und der Deckfläche (116, 126, 136) angeordnet ist. Die Länge (152) des zweiten Bereiches (112) parallel zur Mittelachse (118) ist größer als die Länge (151) des ersten Bereiches (111) parallel zur Mittelachse (118). Der Durchmesser (150) des ersten Bereiches (111) in Bezug auf die Mittelachse (118) in Richtung der Deckfläche (116, 126, 136) nimmt stetig zu und der Durchmesser des zweiten Bereiches (112) in Bezug auf die Mittelachse (118) in Richtung der Deckfläche (116, 126, 136) nimmt stetig ab.

## Beschreibung

### Technisches Gebiet der Erfindung

Die Erfindung betrifft eine Darmrohr-Anordnung zur Applikation einer Flüssigkeit, beispielsweise zur Durchführung von Darmspülungen sowie zum Einbringen von nahrungsoptimierten, probiotischen Bakterienstämmen in den Enddarm eines Lebewesens.

### Hintergrund der Erfindung

Etwa 15 bis 20% der Bevölkerung leiden an erheblichen Problemen bei der Stuhlentleerung. Folgen der mechanischen Behinderung sind ein nagendes Druckgefühl im Unterbauch mit nächtlichen, dumpfen, tiefen Beckenschmerzen durch Kotstauung, gelegentlich tiefen Rückenschmerzen oder linksseitige Inguinalbeschwerden. Kennzeichen der chronischen Verstopfung sind Stuhlschmieren, ein imperativer Stuhldrang und Stuhlentleerungsstörungen, stauungsbedingte, belastende Veränderung der bakteriellen Darmflora, die ein eigenständiges, biologisch aktives Organ im Körper bildet.

Darmspülungen dienen der Behandlung von Störungen des Verdauungstraktes infolge von Fehlernährung, Stress, mangelnder Bewegung und der altersbedingten, verlangsamten Darmperistaltik. Durch den ausgedehnten Aufenthalt des Kots im Darm bilden sich bakterielle Gärungs- und Fäulnissubstanzen, die in den Kreislauf resorbiert werden. Die Selbstvergiftung durch Verstopfung und Völlegefühl hat eine nachhaltige depressionsauslösende Symptomatik auf die Psyche und verursacht beispielsweise Unbehagen, Missstimmung, Burn out und Verdruss.

Die gezielte Entleerung des Enddarmes beseitigt schmerzhafte Begleitbeschwerden, wie Blutstauung mit Hämorrhoiden, Afterjucken und Entzündungen. Die regelmäßige Spülung des Enddarmes mit körperwarmen bis maximal 38°C temperiertem Wasser führt zu einer sicheren Entspannung des Enddarmes. Durch die wärmebedingte Anregung der Peristaltik wird der Darm schonend und sicher entleert. Es können wandständige Skyballa (Kotsteine) entfernt werden. Dadurch wird quälender Juckreiz verhindert sowie die Darmflora und die gesamte Stoffwechselorganisation verbessert. Die regelmäßige Kombination der Darmspülung mit einer Bauchdeckenmassage löst diese Unterbauchschmerzen auf.

Die aktuelle Forschung hat gezeigt, dass die organgleiche Wirkung der Darmflora, die 100 Billionen Bakterien umfasst, - das Genom ist 10mal größer als das Genom des Menschen - unter anderem auf die Psyche, das Immunsystem und den Stoffwechsel Einfluss nimmt und auch eine neurologische Wirkung ausübt. Die Probleme nehmen im Alter zu, die gehemmte Stuhlentleerung verändert die Darmflora in Richtung Fäulnisbakterien. Neben der einfachen Reinigung des Enddarmes zur aboralen Pflege ermöglicht die Darmrohr-Anordnung die Wiederherstellung einer positiv auf den Körper einwirkenden Darmflora mit entsprechender Regulierung des Stuhlverhaltens.

Allgemein bekannt ist die positive Wirkung des probiotischen Joghurts, der im sauren Milieu des Magens stark reduziert wird, und dann nur noch in geringen Mengen das eigentliche Zielgebiet, Dünndarm und oberen Dickdarm, erreicht und von der vorhandenen Darmflora überwuchert wird. Darmspülungen zur Darmsanierung sind eine natürliche Alternative zur oralen Darmsanierung mit Antibiotika oder Abführmitteln. Diese Medikamente sind, da sie, wie auch der Joghurt, den gesamten Magen-Darmtrakt passieren müssen, oft mit unerwünschten Nebenwirkungen verbunden oder werden durch die Einwirkung der Verdauungssäfte in den oberen Darmabschnitten zerstört.

Die positive Dickdarmflora zersetzt auf enzymatischem Weg Unverdauliches oder spendet Vitamin B12. Eine krankmachende Dickdarmflora trägt zur krankhaften Gewichtszunahme ebenso wie zum Typ 2-Diabetes mellitus bei. Die Darmflora hat einen fundamentalen Einfluss auf die Abwehrkräfte. Im Verdauungssystem eines Menschen sind immerhin zwei Drittel seiner Immunzellen angesiedelt. Bei Mäusen ohne Darmflora liegt auch das Immunsystem lahm, auch hat der Darm immerhin 500 Millionen Nervenzellen, deren Bedeutung noch weitestgehend ungeklärt ist.

Die Darmbakterien können im Krankheitsfall eine durchaus negative Wirkung auf Gehirn, Nerven und Psyche entfalten. So zeigten die Fälle von EHEC-Patienten, die bakteriell ausgelöste, schwere, neurologische Probleme entwickelten, die Einwirkung der Dickdarmbakterien auf das Gehirn. Die mit dem Darmrohr mögliche gezielte Beeinflussung der Dickdarmflora lässt eine wirkungsvolle Therapie vermuten: Antibiotika schützen bei Leberversagen durch die Vernichtung der überwuchernden, schädlichen Bakterien vor typischen Begleitsymptomen wie Krampfanfällen, Demenzerscheinungen und komatösen Zuständen. Auch werden Infektionen bei vielen Krankheiten von Alzheimer bis Leberzirrhose als Auslöser oder Mitverursacher vermutet. Dass der Einsatz von gesunden, probiotischen Bakterien auf aboralem Weg eine heilende Wirkung ausübt, zeigt z.B. die Fäkaltransplantation bei Patienten mit lebensgefährlichen Clostridium-Infektionen, die eine Stuhlspende von Gesunden als Zäpfchenweg verabreicht bekommen und überraschend gute Heilergebnisse zeigen. Die Darmflora normalisiert sich in der Regel umgehend, die Zuführung auf aboralen Weg ist zweifelsohne der direktere Weg, da die Magenpassage ausgespart wird.

### Stand der Technik

Das Dokument DE 10 2008 028 221 A1 offenbart ein einfach gekrümmtes Massage- und Stimulationsgerät inklusive Vibrator für das Genitale von Erwachsenen ohne Spülmöglichkeit. Das Dokument DE 1760649 U offenbart ein einfach gekrümmtes Darmrohr zur Applikation von Medikamenten. Das Dokument US 1,253,955 zeigt ein gleichartiges Rohr, dass die Spülmöglichkeit mit einer lokalen Massage der Prostata und einer lokalen elektrischen Reizung ermöglicht. Allerdings ist es mit dem vorgestelltem glatten Darmrohr kaum möglich, dass der doppelte Ringmuskel des Analkanals, da er aus glatter und quergestreifter Muskulatur besteht, schmerzfrei aufgedehnt wird. Zudem hat es sich als nachteilig herausgestellt, dass das Darmrohr mit der Enddarmmuskulatur nicht gehalten werden kann, sondern herausgepresst wird. Außerdem kommt es bei einem glatten Darmrohr zu einem steten Rückfluss der verschmutzten Spülflüssigkeit, die sich über die Hand des Einsetzenden ergießt. Eine separate sterile Zuführung der Spülflüssigkeit unter dem gleichzeitigen Einsatz von Medikamenten bzw. ein bakterieller Besatz sind mit diesem Gerät ausgeschlossen.

Das Dokument DE 91 12 982.6 U1 offenbart eine Vorrichtung zum Einbringen von Spülflüssigkeiten in Körperhöhlungen, bei welcher ein Behälter einen gekrümmten Auslaufstutzen aufweist. Das Dokument DE 203 02404 U1 zeigt einen geraden Einlaufkatheter mit einem kegelförmigen Verschlussteil. Das Dokument AT 100013 B zeigt ein Irrigationsrohr für Darmspülungen mit einfach gekrümmten, außerhalb des Darmes liegenden Teil. Das Dokument AT 119772 B zeigt einen Spülkopf für subaquale Darmspülungen, bei dem das Irrigationsröhrchen außerhalb des Darmes S-förmig nach hinten gebogen ist. Das Dokument CH 132741 A zeigt einen Spülkopf, der an einen Wasserschlauch angeschlossen ist. Der Wasserschlauch wird aus einem wassergefüllten Kissen mit Wasser gespeist. Das Dokument AT 110 651 B zeigt einen eingeführten, gekrümmten Spülkopf, der an einem Gürtel befestigt ist. Das Dokument DE 425141 A zeigt einen Spülkopf in Schlauchform. US 2,606,557 zeigt einen gekrümmten Spülkopf zur Verwendung bei liegenden Personen. Das Dokument DE 298 00 816 U1 offenbart eine Analdusche, die mit einem Schlauch an eine Zapfstelle angeschlossen wird. An einem mit einem handbetätigten Ventil versehenen Handgriff ist ein U-Rohr angeschlossen. Die Analdusche ist nicht zum Einführen in den Darm vorgesehen. Das Dokument US 2007/0299396 A1 offenbart ein Spülapparat für Rachen und Kehle, der aus einem Wassertank über eine Pumpvorrichtung einen Sprühfilm zur Benetzung von Kehle und Rachen, sowie zur Mundreinigung ermöglicht. Das Dokument DE 20 2006 000 696 U1 beschreibt ein doppelwandiges gefülltes Kondom, welches aus einer inneren und einer äußeren Wand besteht, wobei die innere Wand dicht ist und die äußere Wand Löcher enthält, durch die sich zwischen den Wänden befindende Flüssigkeit austreten kann.

Alle zuvor beschriebenen Vorrichtungen, soweit sie überhaupt eine Spülmöglichkeit bieten, ermöglichen nicht die Enddarmspülung bei eigenständiger Handhabung und gleichzeitigem Verschluss der aboralen Körperöffnung.

Weiterhin sind zahlreiche Wassersteckverbindungen zur Anwendung im Gartenbereich bekannt, die allerdings für die kontaminationssichere Zuführung von Flüssigkeiten nicht geeignet sind. Eine Anwendung von Wassersteckverbindungen in Verbindung mit Toilettenspülungen bzw. Toilettenbecken ist bisher nicht beschrieben. Eine Nutzung im Toilettenbereich ist aber im Zusammenhang mit Darmspülgeräten wünschenswert.

Die Handhabung bekannter Darmspülanordnungen ist schwer und umständlich. Eine zusätzliche Zuführung von Reinigungsmitteln bzw. Vorrichtung zur bakteriellen Applikation ist nicht möglich. Dies erfordert einen Schutz gegen Fremdkontamination durch externe Bakterien. Die Benutzung von Darmspülanordnungen durchbricht eine Schamzone der Nutzer. Es ist daher wünschenswert, dass der Nutzer die Anordnung ohne fremde Hilfe anwenden kann. Es ist ferner wünschenswert, dass die Anordnung an die anatomischen Bedingungen angepasst ist.

Wünschenswert sind zudem die Gewährleistung der Hygienesicherheit, das schmerzfreie, sichere Einbringen des Darmrohres und die Sicherstellung einer den Eigenschaften der Schließmuskulatur gerecht werdenden Aufdehnung des Enddarmes.

### Aufgabe der Erfindung und erfindungsgemäße Lösung

Eine erste Aufgabe der vorliegenden Erfindung besteht daher darin, eine gegenüber dem beschriebenen Stand der Technik vorteilhafte Darmrohr-Anordnung zur Applikation einer Flüssigkeit in den Dickdarm eines Lebewesens zur Verfügung zu stellen. Eine zweite Aufgabe besteht darin, ein Griffteil für eine vorteilhafte Darmrohr-Anordnung zur Applikation einer Flüssigkeit in den Dickdarm eines Lebewesens zur Verfügung zu stellen. Eine dritte Aufgabe besteht darin, einen Spülkörper für eine vorteilhafte Darmrohr-Anordnung zur Applikation einer Flüssigkeit in den Dickdarm eines Lebewesens zur Verfügung zu stellen.

Die erste Aufgabe wird durch eine Darmrohr-Anordnung nach Anspruch 1 gelöst. Die zweite Aufgabe wird durch ein Griffteil nach Anspruch 16 gelöst. Die dritte Aufgabe wird durch einen Spülkörper nach Anspruch 17 gelöst. Die abhängigen Ansprüche definieren weitere vorteilhafte Ausgestaltungen der Erfindung. Dabei sind die beschriebenen Merkmale sowohl einzeln, als auch in beliebiger Kombination miteinander vorteilhaft.

Die erfindungsgemäße Darmrohr-Anordnung eignet sich zur Applikation einer Flüssigkeit in den Dickdarm eines Lebewesens, beispielsweise eines Menschen oder eines Tieres. Die Darmrohr-Anordnung umfasst ein Griffteil und einen dem Griffteil aufzusetzenden Spülkörper. Der Spülkörper umfasst einen Fluidkanal. Weiterhin umfasst der Spülkörper mindestens zwei in den Dickdarm eines Lebewesens einführbare Segmente. Jedes Segment umfasst eine Mittelachse, eine Grundfläche, eine Deckfläche, einen ersten Bereich und einen zweiten Bereich. Dabei ist der erste Bereich zwischen der Grundfläche und dem zweiten Bereich angeordnet und der zweite Bereich zwischen dem ersten Bereich und der Deckfläche angeordnet. Die Länge des zweiten Bereiches parallel zur Mittelachse ist größer als die Länge des ersten Bereiches parallel zur Mittelachse. Der Durchmesser des ersten Bereiches nimmt in Bezug auf die Mittelachse in Richtung der Deckfläche stetig, insbesondere kontinuierlich, zu. Der Durchmesser des zweiten Bereiches nimmt in Bezug auf die Mittelachse in Richtung der Deckfläche stetig, insbesondere kontinuierlich, ab.

Die erfindungsgemäße Gestaltung des Spülkörpers ermöglicht ein schmerzfreies, den anatomischen Gegebenheiten des Dick- und Enddarmes gerecht werdendes Einführen des Spülkörpers in den Dickdarm. Vorteilhafterweise kann die konkrete Ausgestaltung der Segmente des Spülkörpers den jeweiligen Gegebenheiten, beispielsweise der Art und dem Alter des Lebewesens, angepasst werden.

Die Länge des ersten Bereiches parallel zur Mittelachse kann insbesondere kleiner als ein Drittel, insbesondere kleiner als ein Viertel oder ein Fünftel, der Länge des zweiten Bereiches parallel zur Mittelachse sein. Weiterhin kann der Durchmesser der Grundfläche mindestens eines Segments zwischen 5% und 15%, insbesondere 10%, geringer sein als der maximale Durchmesser des Segments. Dadurch wird ein sicherer Verschluss des Schließmuskels ermöglicht und ein Herausrutschen oder Herauspressen des Spülkörpers aus dem Darm verhindert.

Die einzelnen Segmente, vorzugsweise können 3 oder 4 Segmente verwendet werden, können miteinander verbunden, insbesondere lösbar verbunden, sein. Dies ermöglicht eine individuelle Zusammensetzung des Spülkörpers entsprechend den jeweiligen Anforderungen.

Alternativ dazu kann der gesamte Spülkörper einstückig ausgestaltet sein. Bei einem einstückig ausgestalteten Spülkörper kann die jeweilige Grundfläche bzw. Deckfläche eines Segments eine gedachte Fläche sein. Eine weitere Möglichkeit besteht darin, die jeweilige Grundfläche bzw. Deckfläche eines Segments zu definieren als die Querschnittsfläche senkrecht zur Mittelachse eines Segments, die den Übergang zwischen zwei benachbarten Segmenten bildet.

Grundsätzlich können die Segmente einen runden oder ovalen Querschnitt aufweisen. Die Querschnittsfläche des ersten Bereiches eines Segments kann in Bezug auf die Mittelachse in Richtung der Deckfläche stetig, insbesondere kontinuierlich, zunehmen. Die Querschnittsfläche des zweiten Bereiches kann in Bezug auf die Mittelachse in Richtung der Deckfläche stetig, insbesondere kontinuierlich, abnehmen.

Zudem kann der Spülkörper ein distales Ende und ein proximales Ende umfassen. Der maximale Durchmesser oder die Querschnittsfläche der Segmente kann in Bezug auf ihre jeweilige Mittelachse vom distalen Ende zum proximalen Ende hin zunehmen. Vorteilhafterweise kann der maximale Durchmesser einander benachbarter Segmente zwischen 20% und 40% voneinander abweichen. Dadurch wird eine sanfte und stufenweise Öffnung und Aufdehnung des Schließmuskels erreicht.

Zudem kann der Spülkörper ein distales Ende umfassen. Zur Gewährleistung eines schmerzfreien Einführens des Spülkörpers kann die Deckfläche des am distalen Ende angeordneten Segments konvex gekrümmt sein.

Das Griffteil oder der Spülkörper können einen nicht in den Darm einführbaren Bereich mit einer Mittelachse umfassen, wobei der nicht in den Darm einführbare Bereich eine Ausdehnung senkrecht zur Mittelachse aufweist, die größer ist als der maximale Durchmesser der Segmente des Spülkörpers. Der nicht in den Darm einführbare Bereich hat die Funktion eines Stoppers und verhindert, dass der Spülkörper zu weit in den Darm eingeführt wird. Zudem schützt er das Griffteil vor einer Verschmutzung durch aus dem Darm heraus fließende Flüssigkeit und Kot.

Der Spülkörper kann ein proximales Ende umfassen und der nicht in den Darm einführbare Bereich kann eine Ausdehnung senkrecht zur Mittelachse aufweisen, die größer ist als der maximale Durchmesser des in Richtung des proximalen Endes letzten Segments des Spülkörpers.

Wie bereits erwähnt, können die Segmente lösbar miteinander verbunden sein. Zusätzlich oder alternativ dazu können das Griffteil und der Spülkörper ebenfalls lösbar miteinander verbunden sein. Dazu kann beispielsweise eine Steckvorrichtung im Bereich der Grundfläche und/oder der Deckfläche der Segmente angeordnet sein. Insbesondere können die Segmente Vorsprünge und/oder Verjüngungen umfassen. Vorteilhafterweise können die Vorsprünge und/oder Verjüngungen so ausgestaltet sein, dass sie zwischen den Mittelachsen zweier benachbarter Segmente einen Winkel zwischen 0° und 80°, insbesondere zwischen 5° und 45°, vorzugsweise zwischen 10° und 30°, bewirken. Dies ermöglicht eine individuelle Zusammensetzung der gesamten Darmrohr-Anordnung aus einer Anzahl unabhängiger Bauteile, die jeweils genormt sein können, womit die konkret benötigte Darmrohr-Anordnung genau an die jeweilige Anwendungssituation angepasst werden kann.

Mindestens ein Segment kann einen im Bereich der Mittelachse des Segments angeordneten Fluidkanal umfassen. Der Fluidkanal kann grundsätzlich innerhalb des Segments und zentrisch oder exzentrisch bezüglich der Mittelachse des Segments angeordnet sein. Weiterhin kann mindestens ein Segment einen Fluidkanal umfassen, der eine Einlassöffnung und eine Auslassöffnung umfasst. Die Einlassöffnung und/oder die Auslassöffnung können an der Grundfläche des Segments im Bereich der Mittelachse des Segments angeordnet sein. Die Einlassöffnung und/oder die Auslassöffnung kann an der Deckfläche des Segments in radialer Richtung von der Mittelachse des Segments beabstandet angeordnet sein. Vorteilhafterweise ist die Einlassöffnung an der Grundfläche des Segments, insbesondere des Segments am distalen Ende des Spülkörpers, im Bereich der Mittelachse des Segments angeordnet und die Auslassöffnung an der Deckfläche des Segments in radialer Richtung von der Mittelachse des Segments beabstandet angeordnet. Die ermöglicht eine effektive Darmwandspülung.

Die Grundfläche und die Deckfläche mindestens eines Segments können jeweils eine Flächennormale aufweisen, wobei die Flächennormale der Grundfläche mit der Flächennormale der Deckfläche einen Winkel zwischen 0° und 80°, beispielsweise zwischen 5° und 45°, vorzugsweise zwischen 10° und 30°, einschließt. Hierdurch lässt sich eine an die anatomischen Gegebenheiten angepasste Form der Segmente realisieren.

Abweichend von der üblichen Konvention, nach welcher die Flächennormale einer Oberfläche nach außen gerichtet ist, wird im Rahmen der vorliegenden Erfindung die Flächennormale der Grundfläche als nach innen gerichtet definiert. Die Flächennormale der Deckfläche wird dagegen im Rahmen der vorliegenden Erfindung konventionell als nach außen gerichtet definiert. Bei der jeweiligen Grund- bzw. Deckfläche kann es sich grundsätzlich um eine Grund- bzw. Deckfläche eines Segments oder eines Verjüngungsbereiches handeln.

Weiterhin kann der Spülkörper mindestens einen Verjüngungsbereich umfassen, der zwischen zwei Segmenten angeordnet sein kann und der eine Grundfläche mit einer Flächennormale und eine Deckfläche mit einer Flächennormale umfasst. Dabei kann die Flächennormale der Grundfläche mit der Flächennormale der Deckfläche einen Winkel zwischen 0° und 80°, beispielsweise zwischen 5° und 45°, vorzugsweise zwischen 10° und 30°, einschließen. Auf diese Weise lassen sich beispielsweise bezüglich ihrer Mittelachse symmetrische Segmente verwenden, wobei eine Krümmung des Spülkörpers durch entsprechende, die Verjüngungsbereiche bildende Zwischenstücke erreicht werden kann. Die Verjüngungsbereiche können also separate Bauteile sein, die mit den Segmenten verbunden, insbesondere lösbar verbunden sind.

Das Griffteil kann eine Mittelachse umfassen. Der Spülkörper kann ein proximales Ende und eine Mittelachse im Bereich des proximalen Endes umfassen. Die Mittelachse des Griffteils kann mit der Mittelachse des Spülkörpers im Bereich des proximalen Endes einen Winkel zwischen 50° und 90°, vorzugsweise zwischen 65° und 75°, einschließen. Dies ermöglicht eine bequeme Handhabung durch den Nutzer.

Grundsätzlich kann das Griffteil und/oder der Spülkörper eine Vorrichtung zum Einbringen einer Flüssigkeit in den Fluidkanal des Spülkörpers umfassen und/oder mit einer Vorrichtung zum Einbringen einer Flüssigkeit in den Fluidkanal verbunden, insbesondere strömungstechnisch verbunden, sein. Unter einer Flüssigkeit wird im Rahmen der vorliegen Erfindung auch eine aufgeschäumte Flüssigkeit bzw. ein auf einer zumindest teilweise flüssigen bzw. einer gelförmigen Substanz basierender Schaum verstanden. Die zu applizierende Flüssigkeit kann also auch in Form von Schaum appliziert werden.

Bei der Vorrichtung zum Einbringen der Flüssigkeit in den Fluidkanal des Spülkörpers kann es sich also zum Beispiel um eine Flüssigkeitszufuhr oder einen Schaumapplikator handeln. Dazu kann das Griffteil einen Fluidkanal, der strömungstechnisch mit dem Fluidkanal des Spülkörpers verbunden ist, umfassen. Zudem kann das Griffteil ein Ventil zur Freigabe einer Fluidströmung, also beispielsweise der zu applizierenden Flüssigkeit oder des Schaums, umfassen. Bei dem Ventil kann es sich um ein in das Griffteil integriertes Steuerventil, z.B. ein Schiebeventil oder ein Hebelventil, handeln. Weiterhin kann das Griffteil einen Luer-Lockanschluss, insbesondere zum Zuführen von Reinigungsmitteln oder anderen Zusätzen, vorzugsweise Glycerin und/oder Elektrolyte, umfassen.

Bei dem zu applizierenden Fluid bzw. der Flüssigkeit kann es sich zum Beispiel um Wasser, eine Reinigungslösung, Glycerin und/oder Elektrolyte, Joghurt, insbesondere versetzt mit Bakterienstämmen wie zum Beispiel Lactobacillus bulgaricus und Streptococcus thermophilus, handeln.

Darüber hinaus kann der Spülkörper einen Aufsatz umfassen, welcher die äußere Oberfläche mindestens eines Segmentes zumindest teilweise bedeckt. Der Aufsatz kann als Schutzkondom ausgestaltet sein, welches die äußere Oberfläche mindestens eines Segmentes zumindest teilweise bedeckt. Weiterhin kann der Aufsatz ein Reservoir, beispielsweise einen Hohlraum oder ein Gleitmittelreservoir, umfassen. Der Aufsatz bzw. das Schutzkondom ist vorzugsweise doppelwandig ausgestaltet, also mit einer äußeren Wand oder Hülle und mit einer inneren Wand oder Hülle, wobei die innere Wand/Hülle vorteilhafterweise stabiler ausgestaltet ist als die äußere Wand/Hülle. Der Aufsatz bzw. das Schutzkondom kann grundsätzlich Silikon und/oder eine wasserlösliche Folie, vorzugsweise eine kaltwasserlösliche Folie, z.B. Polyvinylalkohol (PVAL), umfassen. Der Aufsatz bzw. das Schutzkondom kann einen runden oder ovalen Querschnitt haben.

Der Spülkörper kann grundsätzlich alle Materialen umfassen bzw. aus allen Materialien bestehen, bei denen keine Zytotoxizität nach Transfer mit Fibroblasten und keine Hautreizung nach Transfer mit Keratinozyten besteht. Vorzugsweise ist das Material frei von Phthalaten und antiallergisch. Als Basismaterial für den Spülkörper empfiehlt sich Silikon in seinen unterschiedlichen Elastizitätsgraden, die eine besonders gute Körperverträglichkeit zeigen. Es sind aber auch alle anderen formbaren Materialien denkbar, da Gewebsunverträglichkeiten sich durch ein aufgesetztes antiallergisches Schutzkondom immer beherrschen lassen. Metalle haben den Vorteil der besseren Sterilisierbarkeit und zeichnen sich durch eine lange Lebensdauer aus, Korrosionsfreiheit vorausgesetzt.

Das erfindungsgemäße Griffteil eignet sich für eine Darmrohr-Anordnung zur Applikation einer Flüssigkeit in den Dickdarm eines Lebewesens, insbesondere für eine zuvor beschriebene Darmrohr-Anordnung. Das erfindungsgemäße Griffteil umfasst eine Vorrichtung zur Steuerung der Zufuhr der zu applizierenden Flüssigkeit in einen Fluidkanal der Darmrohr-Anordnung. Das erfindungsgemäße Griffteil hat dieselben Eigenschaften und Vorteile wie das Griffteil der zuvor beschriebenen erfindungsgemäßen Darmrohr-Anordnung. Die bereits im Zusammenhang mit dem Griffteil der Darmrohr-Anordnung beschriebenen Merkmale können auch Merkmale des erfindungsgemäßen Griffteils sein.

Insbesondere kann das Griffteil einen Luer-Lock-Anschluss zur Medikamenteneinbringung umfassen. Es kann auswechselbar sein und unterschiedliche Funktionen übernehmen, mit einem Darmspülrohr verbindbar ausgestaltet sein und den anatomischen Gegebenheiten in Größe und Länge (Kleintiere, Großtiere, Kind, Erwachsener etc.) angepasst werden. Das Griffteil kann zudem durch einen Schaumapplikator ersetzt werden oder einen Schaumapplikator umfassen, mit dem sich ebenso wie über den Luer-Lock-Anschluss des Griffteiles joghurtähnliche, probiotische Bakterienstämme einführen lassen, die einen positiven Einfluss auf die Darmbesiedelung haben.

Der erfindungsgemäße Spülkörper eignet sich für eine Darmrohr-Anordnung zur Applikation einer Flüssigkeit in den Dickdarm eines Lebewesens, insbesondere für eine zuvor beschriebene Darmrohr-Anordnung. Der Spülkörper umfasst einen Fluidkanal. Weiterhin umfasst der Spülkörper mindestens zwei in den Dickdarm eines Lebewesens einführbare Segmente. Jedes Segment umfasst eine Mittelachse, eine Grundfläche, eine Deckfläche, einen ersten Bereich und einen zweiten Bereich. Der erste Bereich ist zwischen der Grundfläche und dem zweiten Bereich angeordnet und der zweite Bereich ist zwischen dem ersten Bereich und der Deckfläche angeordnet. Die Länge des zweiten Bereiches ist parallel zur Mittelachse größer als die Länge des ersten Bereiches parallel zur Mittelachse. Der Durchmesser des ersten Bereiches nimmt in Bezug auf die Mittelachse in Richtung der Deckfläche stetig zu und der Durchmesser des zweiten Bereiches nimmt in Bezug auf die Mittelachse in Richtung der Deckfläche stetig ab. Der erfindungsgemäße Spülkörper hat dieselben Eigenschaften und Vorteile wie der Spülkörper der zuvor beschriebenen erfindungsgemäßen Darmrohr-Anordnung. Die bereits im Zusammenhang mit dem Spülkörper der Darmrohr-Anordnung beschriebenen Merkmale können auch Merkmale des erfindungsgemäßen Spülkörpers sein.

Die erfindungsgemäße Darmrohr-Anordnung ermöglicht eine stete aborale Pflege des Enddarmes und die Einbringung positiv wirkender Bakterienstämme, z.B. Lactobacillus bulgaricus und Streptococcus thermophilus. Allgemein bekannt ist die positive Wirkung des probiotischen Joghurts, dass im sauren Milieu des Magens stark reduziert wird, und dann nur noch in geringen Mengen das eigentliche Zielgebiet, Dünndarm und oberen Dickdarm, erreicht und von der vorhandenen Darmflora überwuchert wird. Darmspülungen zur Darmsanierung sind eine natürliche Alternative zur oralen Darmsanierung mit Antibiotika oder Abführmitteln. Diese Medikamente sind, da sie, wie auch der Joghurt, den gesamten Magen-Darmtrakt passieren müssen, oft mit unerwünschten Nebenwirkungen verbunden oder werden durch die Einwirkung der Verdauungssäfte in den oberen Darmabschnitten zerstört.

Die erfindungsgemäße Darmrohr-Anordnung, insbesondere zur Durchführung von Darmspülungen, kann einfach, doppelt bzw. dreifach gekrümmt ausgestaltet sein, wobei es vom Nutzer selber angewendet werden kann, den anatomischen Gegebenheiten angepasst und schmerzfrei zu handhaben und einfach aufgebaut ist.

Die Segmente des Spülkörpers können eine geschossartige Form mit ansteigendem Durchmesser haben. Die Verbindung zwischen dem Spülkörper und dem Griffteil, sowie zu einem an den Griff anschließbarem Schlauchsystem kann über einen Doppelsteckanschluss erfolgen, der eine kontaminationssichere Flüssigkeitszufuhr ermöglicht. Zum Beispiel kann eine wandständige Wassersteckdose mit einer gleichartigen kontaminationssicheren Verbindung ausgestattet werden, die einen Anschluss an eine normale Warmwasserzufuhr ermöglicht.

Bei der Anwendung der erfindungsgemäßen Darmrohr-Anordnung kann der Spülkörper oder Spülkörper-Aufsatz mit dem außerhalb des Darmes vorgesehenen Griffteil von der Bauchseite kommen, den Spülkörper-Aufsatz zwischen den Beinen nach hinten in die aborale Körperöffnung eingeführt werden. Dies ermöglichen der Knick zwischen dem Griffteil und dem Spülkörper und eine Krümmung des Spülkörpers. Der Spülkörper bzw. dessen Segmente sind so gestaltet, dass der Spülkörper an dem in den Darm einzuführenden Teil eine weitere Krümmung nach vorn und für den humanen Gebrauch, den anatomischen Ano-Rektumverlauf berücksichtigend, auch zur linken Seite hin aufweist.

Mit der außerhalb des Darmes liegenden Krümmung bzw. dem Knick ist es möglich das Griffteil vor dem Körper zu halten. Der Winkel beträgt vorzugsweise 50 bis 90°, besonders bevorzugt 65 bis 75°. Dadurch kann die Darmrohr-Anordnung besser eingeführt und gehalten werden. Anders als bei einigen Ausführungen nach dem Stand der Technik wird hier kein Gürtel oder sonstige starre Haltevorrichtung vorgesehen. Dadurch, dass die Darmrohr-Anordnung in der Hand gehalten wird, und die beschriebenen Krümmungen hat, wird vermieden, dass der Spülkörper eine falsche, möglicherweise verletzende oder schmerzhafte Lage einnimmt.

Mit der beschriebenen Darmrohr-Anordnung können die eingangs beschriebenen Probleme weitgehend gelöst werden. Die insbesondere altersbedingte, verlangsamte Nahrungs- und Stuhlpassage durch den Darm kann mit der Darmspülung unterstützt werden. Kotsteinbildung wird vermieden bzw. kann bei regelmäßiger Nutzung gar nicht mehr auftreten. Das Griffteil ähnelt in seiner Form vorzugsweise einem Quader mit Vertiefungen für die Langfinger mit einer aufgesetzten Abwinkelung für das aufzusetzende Spülrohr auf der einen Seite, so dass es gut in der Hand liegt und sich problemlos vom Nutzer führen lässt. Die Winkelung des Griffteils ist dem Nutzer anzupassen; denn es ist von erheblicher Bedeutung, ob die Person, die das Griffteil führt die Spülung an sich selbst oder an einem anderen Lebewesen durchführt. Wird das Darmrohr zur Spülung eines stehenden Lebewesens, z.B. eines Rindes benutzt, so ist ein Winkel von bis zu 145° - 170° sinnvoll, um den Darm rundum zu spülen, ganz anders wenn es sich um einen sitzenden von vorn sich selbst spülenden Menschen handelt; dann sind eher Winkelgrade von 100° - 145° angezeigt.

Die Flüssigkeitszufuhr kann idealerweise über die Basis des Quaders mit seinen Vertiefungen, entweder über ein Schraub- oder über ein Steckgewinde, dass in seinem Durchmesser den Dimensionen des Spülrohres angepasst ist, erfolgen. Eine seitliche Zufuhr zum Beispiel über den winkelseitigen Handgriff ist aber auch durchaus möglich. Die definitive Wasserzufuhr kann über ein integriertes Steuerventil, z.B. ein Schiebeventil oder ein Hebelventil reguliert werden. Wird die Wasserzufuhr bereits vorher reguliert, so kann das Ventil entfallen. Ist das Ventil integriert, so reguliert der Nutzer die Flüssigkeitszufuhr über den Schiebeschalter bzw. Hebel im Griffteil. Das Ventil sollte gleichzeitig einen Rückfluss des Darminhaltes in die zugeführte Flüssigkeit verhindern. Dadurch ist dem Nutzer jederzeit eine Feinregulation der letztendlichen Wasserzufuhr ermöglicht. Dies setzt allerdings einen erfahrenden Nutzer voraus, bei dem durch eine ärztliche Untersuchung sicher gestellt ist, dass keine Gefährdungen durch Darmwandschwächen wie z.B. Dickdarmdevertikel erfolgen können.

Über das Griffteil kann eine zusätzliche Flüssigkeitszufuhr über einen Luer-Lock-Anschluss hergestellt werden, falls z. B. bei speziellen Darmproblemen ergänzende Substanzen, Naturheilmittel zur Lösung und Aufweichung des Darminhaltes zusammen mit der Spülflüssigkeit eingebracht werden sollen.

Der abgewinkelte Griff kann mit einer Schutzplatte enden, die in ihrem Umfang der Handgröße des Nutzers angepasst werden kann. Die Flüssigkeitszufuhr, insbesondere die Wasserzufuhr kann über unterschiedliche Steckverbindungen erfolgen.

In einer weiteren Ausgestaltung der Erfindung ist die Wasserversorgung aus einem Reservoir mit begrenztem Volumen gebildet oder es ist ein Reservoir mit begrenztem Volumen zwischen der Wasserversorgung und dem Griffteil vorgesehen. Ein solches Reservoir kann beispielsweise von einer Blasenspritze gebildet sein. Das Reservoir sollte bei Darmrohr-Anordnungen zur Darmspülung von Erwachsenen ein Volumen von 200 ml nicht übersteigen. Mit dem Reservoir wird verhindert, dass zu viel Wasser in den Darm gelangt und Verletzungen vorgebeugt.

In einer besonders bevorzugten Ausgestaltung kann das Griffteil auch als Behältnis für die Spülflüssigkeit, die dann z.B. über ein Treibmittel in den Darm eingebracht werden kann, als Schaumapplikator benutzt werden. Dies ermöglicht dann eine von einer Wasserzufuhr völlig unabhängige Nutzung, erlaubt in der Regel aber nur eine sehr begrenzte Flüssigkeitszufuhr in den Enddarm. Das Flüssigkeitsvolumen aufgeschäumt ist für den gewünschten Zweck durchaus ausreichend.

Das Griffteil kann auch durch einen Schaumapplikator zur Beschichtung der Darmoberfläche mit einem bakterientragenden aufgeschäumten Selektiv- oder Minimalnährmedium ersetzt werden. Hierfür können nur sterile Einmalprodukte verwendet werden, damit es keine Verschmutzungen der gewünschten Nährmedien gibt, um die gewünschte individuelle Wirkung sicher zu erzielen. Dieses Verfahren hat den Vorteil, dass man entsprechende Selektivmedien einsetzen kann, die eine optimale Zusammensetzung der Bakterienkulturen garantieren.

Mit dem abgewinkelten Griffteil/Schaumapplikator soll die Flüssigkeitszufuhr für das Spülrohr hergestellt, die Rundumspülung des Darmes zur Ablösung fester Stuhlreste erleichtert werden bzw. eine gleichmäßige Verteilung der joghurtähnlichen bakteriellen Kulturen (Joghurt ist z.B. die optimale Trägersubstanz für Lactobacillus bulgaricus und Streptococcus thermophilus) für die orale Bakterienzuführung. Mit dem integriertem Steuerventil bzw. durch den Einmalschaumapplikator wird sicher gestellt, dass der Nutzer jederzeit die Wasserzufuhr regulieren bzw. steuern und abschalten kann, falls es z.B. zu Verspannungsgefühlen kommt. Eine Zufuhr von Flüssigkeiten über einen getrennten Zulauf im Griffteil (z.B. Luer-Lock) kann integriert werden.

Für die Darmspülung ist der Nutzer nicht mehr auf Fremdhilfe angewiesen, sondern kann die Darmreinigung ungestört verrichten. Durch das zusätzliche Ventil wird für den Nutzer ein noch höherer Sicherheitsgrad erreicht und es ermöglicht das Darmspülrohr auch an einen Warmwasseranschluss direkt anzubinden. Das Griffteil bzw. der Schaumapplikator ermöglichen erst die problemlose Nutzung des Darmspülrohres, ist also ein Teil des Gesamtkonzeptes. Bei aufgesetztem Griffteil hat der Spülkörper bzw. Spülkörperaufsatz folgende wesentliche Funktionen: die Flüssigkeitszufuhr, die Lenkung des über das gekrümmte Darmrohr abgegebenen Flüssigkeitsstrahl, die Aufdehnung des Schließmuskels, durch die Segmentierung den Verschluss der Darmöffnung gegen den seitlichen Rückfluss der Spülflüssigkeit aus dem Darm, und Gewährleistung eines sicheren Haltes der Spülkörperanordnung während der freihändigen Nutzung.

In einer weiteren Ausgestaltung der Erfindung umfasst der in den Darm einzuführende Spülkörperaufsatz der Darmrohr-Anordnung mehrere, vorzugsweise drei Teilabschnitte, zwischen denen Verjüngungen gebildet sind. Der Spülkörperaufsatz kann mit einem Stopper enden. Mit dem Stopper wird verhindert, dass der Spülkopf-Aufsatz zu weit in den Darm eingeführt wird und möglicherweise Verletzungen hervorruft. Der Stopper kann von einer konisch zulaufenden Manschette oder einem Rand gebildet sein. Vorzugsweise ist der Stopper an den letzten Teilabschnitt des in den Darm einzuführenden Teils der Darmrohr-Anordnung bzw. an den Spülkörper-Aufsatz angeformt.

Die Teilabschnitte oder Segmente können aus einem Stück aber auch zusammengesteckt sein. Zu diesem Zweck können Vorsprünge und Verjüngungen in den Teilabschnitten vorgesehen sein. Die Verjüngungen dienen der Entspannung der nacheinander geschalteten Verschlussmuskeln, die jetzt die Darmrohranordnung festhalten können, so dass der Nutzer loslassen und die Hände frei hat, sowie zur weiteren Sicherung des gas- und flüssigkeitsdichten Verschlusses.

Der Durchmesser der Teilabschnitte kann zum darmseitigen Ende hin abnehmen, wobei die Segmente vom Darm zum Griffteil jeweils einen Durchmesserzuwachs um 20% - 40% erfahren. Dann wird zunächst der kleinste Teil eingeführt. Die einzelnen Kanalabschnitte des Spülkörper mit ihrer eichel- oder geschossartigen Form passen sich ideal der Schließmuskelfunktion an: Eine langsame, sanfte, stetige Dehnung ist die Voraussetzung, dass ein glatter Muskel sich öffnet, sonst verkrampft er und lässt sich nur unter sehr unangenehmen Qualen lösen. Die schmerzfreie Öffnung des Schließmuskels ist Voraussetzung dafür, dass der Spülkörper einsetzbar ist. Dazu können folgende Maßnahmen vorgesehen sein: Entkrampfung mit einem Nitropräparat, dem Gleitmittel, eichel- oder geschossartige Form des Spülkörpers und/oder ein steiler Abfall zu der Verjüngung zwischen den Einzelsegmenten. Im Querschnitt können die Segmente oval oder rund sein. Der Vorteil des ovalen Spülkörpers liegt in der besseren Berücksichtigung der Muskelanatomie. Bei ovalem Spülkörper wird die Aufhängung des unteren Beckenbodens zwischen der Spitze des Kreuzbeines und dem vorderen Beckenkamm, die in sagittaler Ausrichtung aufgespannt sind, berücksichtigt. Selbstverständlich ist auch eine andere Form möglich z.B. eiförmig etc. Die Form wird der Nutzer selbst bestimmen, da der Beckenboden mit zunehmendem Alter erschlafft, so dass sich die Bedingungen für den Nutzer ändern. Insbesondere die glatte Muskulatur des inneren Verschlussmuskels lässt sich schmerzfrei nur stufenweise öffnen, da der Nutzer die Spannung der glatten Muskulatur nicht willentlich beeinflussen kann. Sobald warme Flüssigkeit in den Enddarm gelangt, erschlaffen die Enddarmverschlussmuskeln (Sphinkter ani ex- und internus). Die Enddarmöffnung bleibt bei der erfindungsgemäßen Anordnung beim Nachschieben der Darmrohr-Anordnung verschlossen. Eine Beschmutzung des Nutzers wird so vermieden. Die beiden Enddarmverschlussmuskeln, Analsphinkter, können sich mit dem Plexus haemorrhoidales im Bereich der Verjüngungen zwischen den Teilabschnitten anschmiegen, so dass ein sicherer, schmerzfreier Verschluss erreicht wird. Zur Entfernung wird der Spülkörper leicht gekippt um ihn zu lösen.

In einer besonders bevorzugten Ausgestaltung der Erfindung weist der in den Darm einzuführende Teil im Inneren einen flüssigkeitsführenden Kanal auf, dessen darmseitige Mündung entweder zentrisch oder exzentrisch auf der der Krümmungsachse zugewandten Seite angeordnet ist. Dadurch wird die Rundumspülung innerhalb des Darmes durch Drehen erleichtert. Der Spülkanal sollte an der Unterseite, also dem Zentrum der Krümmung näher, geführt werden, da diese stets der Darmwand näher liegt, also den Stuhl gezielt ablöst. Aber auch eine zentrierte Führung ist durchaus möglich um den Darminhalt aufzuschwemmen, allerdings ist die direkte Darmwandreinigung damit erschwert, so lange der Darm stuhlgefüllt ist. Eine Führung auf der Oberseite bzw. der übrigen Zirkumferenz sind durchaus möglich, wenn eine direkte Einwirkung auf die zu lösende bzw. zu beeinflussende Stuhlmasse erwünscht ist. Ein hyperbelartiger Längsschnitt der Einzelsegmente ermöglicht es, durch einfache Kippung der Darmrohranordnung den Spülkörper aus der muskulären Haltung zu lösen.

Da die Darmentleerung nicht alleine hinsichtlich der besseren Gleitfähigkeit beeinflusst wird, sondern der aufgesetzte Spülkörper mit seiner Dehnungswirkung auf den Schließmuskel, einen wesentlichen Beitrag zur Enddarmentleerung hat, kann das gewünschte Ziel auch mit relativ geringen Flüssigkeitsmengen schon erreicht werden.

Der beschriebene Spülkörper ist grundsätzlich für alle Lebewesen, also sowohl bei Tieren, wie bei Menschen einsetzbar. Hierzu sind die entsprechenden Dimensionierungen artgerecht anzupassen. Da mit der Größe des Lebewesens auch der Schließmuskel des Darms an Volumen zunimmt wird auch hier die Länge der Verjüngung proportional zum Durchmesser der Segmente zunehmen. Der Durchmesser wird der Größe der Lebewesen, für die es eingesetzt wird, angepasst. Der Spülkörper sollte im Durchmesser größer sein, als die Öffnung des Spülkanals. Durch die Öffnung des Spülkanals lässt sich die zugeführte Flüssigkeitsmenge pro Zeiteinheit ebenso regulieren und auch der Druck, der auf die Darmwand einwirkt. Dies ist der Art des Lebewesens entsprechend zu dimensionieren.

Als Material für den Spülkörper kommen grundsätzlich alle Materialien in Betracht, bei denen keine Zytotoxizität nach Transfer mit Fibroblasten und keine Hautreizung nach Transfer mit Keratinozyten besteht. Vorzugsweise ist das Material frei von Phthalaten und antiallergisch. Als Basismaterial für den Spülkörper empfiehlt sich Silikon in seinen unterschiedlichen Elastizitätsgraden, die eine besonders gute Körperverträglichkeit zeigen. Es sind aber auch alle anderen formbaren Materialien denkbar, da Gewebsunverträglichkeiten sich durch ein aufgesetztes antiallergisches Schutzkondom immer beherrschen lassen. Metalle haben den Vorteil der besseren Sterilisierbarkeit und zeichnen sich durch eine lange Lebensdauer aus, Korrosionsfreiheit vorausgesetzt.

Der wesentliche Vorteil gegenüber Vergleichsprodukten ist der relativ geringe Materialaufwand und die Handhabbarkeit ohne jede fremde Hilfe. Allerdings lässt sich das Darmrohr bei entsprechender Änderung des Winkels zwischen Griffteil und Spülkörper auch durchaus zur Darmspülung von Bettlägerigen und Kranken durch Pflegepersonal benutzen, allerdings ist dies bei der Gestaltung des Winkels zwischen Griffteil und Spülkörper zu beachten, der entsprechender flacher sein muss.

Eine vorteilhafte Schlauchverbindung für die Flüssigkeitszufuhr besteht zum Beispiel aus einem Stecker und einer Muffe, die den Schlauch verbinden. Um eine kontaminationssichere Steckverbindung herzustellen, besteht diese vorzugsweise aus einer zentralen Steckverbindung mit einem Konus als Muffe und einer einfachen Stecköffnung als Gegenstück. Der Stecker besteht zum Beispiel aus einem Rohrsegment mit überworfenem Gummiring. Das Gegenstück die Muffe besteht zum Beispiel aus einem Rohrsegment, das mit einem Silikonring abschließt und einem Überwurfring. Die Schlauchverbindungen dienen der Flüssigkeitszufuhr. Die Muffe kann als Wassersteckdose in einen Festkörper z.B. Wasserbecken, Toilettenbecken oder den Boiler integriert werden, während die Steckverbindung in der Regel am freien Schlauchende befestigt ist. Der Vorteil der Schlauchverbindung ist die problemlose Anwendung der aufsteckbaren Funktionskörper im gesamten durch die Schlauchlänge bestimmten Bereich. Der Schlauch lässt sich nach Nutzung problemlos lagern und jederzeit in Betrieb nehmen. Die Verbindung mit einer wandständigen Wassersteckdose bzw. eine Integration der Wasserzufuhr in ein Toilettenbecken hat den Vorteil, dass auf diese Weise Schlauchverbindungen zum Einsatz der verschiedenen Gerätschaften kurz gehalten werden können, so dass der Schlauch den Nutzer nicht behindert.

Insbesondere kann die Darmrohr-Anordnung zur Durchführung von Darmspülungen ein an eine Flüssigkeitsversorgung anschließbares Griffteil, und einen an das Griffteil anschließbaren oder verbundenen und mit einem vorderen Teil zumindest teilweise in den Darm einzuführenden Spülkörper-Aufsatz enthalten, wobei das Griffteil der der Spülkörper-Aufsatz an dem außerhalb des Darmes vorgesehenen Teil eine, die Führung des Spülkörper-Aufsatzes von vorne ermöglichende Krümmung oder einen Knick aufweist, und der Spülkörper-Aufsatz an dem in den Darm einzuführenden Teil eine weitere Krümmung aufweist. Der Spülkörper-Aufsatz kann abtrennbar sein und/oder als Einwegartikel ausgebildet sein.

Ein Schutzkondom mit integriertem Gleitmittelreservoir, aus welchem Gleitmittel, vorzugsweise Salbe, weiter bevorzugt mit einem Betäubungsmittel versetzte Salbe, kann auf den Spülkörper-Aufsatz aufgezogen werden, wobei das Gleitmittel beim Einführen in den Darm austritt.

Der in den Darm einzuführende Teil der Darmrohr-Anordnung kann mehrere, vorzugsweise drei Teilabschnitte umfassen, zwischen denen Verjüngungen gebildet sind. Die Größe der Teilabschnitte zum darmseitigen Ende hin abnimmt. Die Länge der Teilabschnitte kann proportional zum Durchmesser der Segmente zunehmen. Der in den Darm einzuführende Teil der Darmrohr-Anordnung kann im Inneren einen flüssigkeitsführenden Kanal aufweisen, dessen darmseitige Mündung exzentrisch auf der der Krümmungsachse zugewandten Seite angeordnet ist. Ein Stopper kann vor dem in den Darm einzuführenden Teil der Darmrohr-Anordnung angeordnet sein.

Eine Wasserversorgung, die von einem Reservoir mit begrenztem Volumen gebildet ist, oder ein Reservoir mit begrenztem Volumen kann zwischen der Wasserversorgung und dem Griffteil vorgesehen sein. An dem Griffteil kann ein Ventil zur Freigabe einer Wasserströmung vorgesehen sein. Auf der dem Darm abgewandten Seite des Griffteils kann ein Luer-Lockanschluss zum Zuführen von Reinigungsmitteln oder anderen Zusätzen, vorzugsweise Glycerin und/oder Elektrolyte vorgesehen sein. Das Griffteil kann mit integriertem Schaumgenerator als Einwegartikel ausgebildet sein, der die Verwirbelung der Flüssigkeitszufuhr mit einer selektiven bakteriellen Nährlösung zur Zuführung probiotischer, die Darmtätigkeit besonders fördernde Bakterienstämme ermöglicht.

Eine Krümmung des Spülkopf-Aufsatzes innerhalb des Darmes in Verbindung mit dem Griffteil ermöglicht eine Drehung der Darmrohr-Anordnung und somit eine Rundumspülung innerhalb des Darmes. Das Spülungsergebnis ist besser als bei bekannten Anordnungen. Vorzugsweise umfasst der in den Darm einzuführende Teil der Darmrohr-Anordnung einen abtrennbaren, gekrümmten Spülkörper-Aufsatz. Das hat den Vorteil, dass der Spülkörper-Aufsatz leicht zu reinigen und zu desinfizieren ist. Der Spülkörper-Aufsatz kann insbesondere auch als Einwegartikel ausgebildet sein. Dann sind besonders hygienische Bedingungen gewährleistet. Der Spülkörper-Aufsatz kann mit einem weichen Material, beispielsweise Silikon, überzogen sein.

In einer besonders bevorzugten Ausgestaltung der Erfindung ist ein Gleitmittelreservoir am darmseitigen Ende der Darmrohr-Anordnung vorgesehen, aus welchem Gleitmittel, vorzugsweise Salbe, weiter bevorzugt mit einem Betäubungsmittel versetzte Salbe, beim Einführen in den Darm austritt. Das Gleitmittelreservoir kann beispielsweise von einer Kappe oder einer Blase gebildet sein, die sich beim Einführen des Darmrohres öffnet. Dann tritt das Gleitmittel aus und erleichtert das Einführen. In einer besonders bevorzugten Ausgestaltung der Erfindung ist das Gleitmittel eine mit einem Betäubungsmittel versehene Salbe. Bei Kontakt der Salbe wird der Schließmuskel durch das Betäubungsmittel entspannt und das Einführen weiter erleichtert. Dies ist besonders bei Patienten, die aufgrund von Hämorrhoiden und Entzündungen sehr schmerzempfindlich sind, sinnvoll.

Das Gleitmittelreservoir ist vorzugsweise doppelwandig ausgebildet. Es besteht aus einer kräftigeren, d.h. weniger zerreißenden Rückwand und einer dünneren Vorderwand mit einem aufgesetzten Zentrierkörper aus einem glukosehaltigem oder anderem wasserlöslichem Produkt. Das glukosehaltige Produkt kann sich später im Darm auflösen. Der Zentrierkörper zerreißt beim Aufsetzen des Spülrohres, so dass das Gleitmittel frei wird.

In einer weiteren Ausgestaltung der Erfindung umfasst der in den Darm einzuführende Teil der Darmrohr-Anordnung mehrere, vorzugsweise drei Teilabschnitte, zwischen denen Verjüngungen gebildet sind. Die Teilabschnitte können zusammengesteckt sein. Zu diesem Zweck können Vorsprünge und Verjüngungen in den Teilabschnitten vorgesehen sein. Die Vorsprünge und Verjüngungen können eine Form haben, mit welcher die Krümmung des zusammengesetzten Teils der Darmrohr-Anordnung erreicht wird.

Vorzugsweise nimmt die Größe der Teilabschnitte zum darmseitigen Ende hin ab. Dann wird zunächst der kleinste Teil eingeführt. Die Form ist so gestaltet, dass sich der primär kontrakte Schließmuskel bei der Anwendung der Anordnung bei der Spülung schrittweise öffnet. Sobald warme Flüssigkeit in den Enddarm gelangt, erschlafft der Enddarmverschlussmuskel (Sphinkter ani ex- und internus). Die Enddarmöffnung bleibt bei der erfindungsgemäßen Anordnung beim Nachschieben der Darmrohr-Anordnung verschlossen. Eine Beschmutzung des Nutzers wird so vermieden. Die beiden Analsphinkter können sich mit dem Plexus haemorrhoidales im Bereich der Verjüngungen zwischen den Teilabschnitten anschmiegen, so dass ein sicherer, schmerzfreier Verschluss erreicht wird.

In einer besonders bevorzugten Ausgestaltung der Erfindung weist der in den Darm einzuführende Teil im Inneren einen flüssigkeitsführenden Kanal auf, dessen darmseitige Mündung exzentrisch auf der der Krümmungsachse zugewandten Seite angeordnet ist. Dadurch wird die Rundumspülung innerhalb des Darmes durch Drehen erleichtert.

Die einzelnen Segmente des Spülköpers mit einer eichel- oder geschossartigen Form passen sich ideal der Schließmuskelfunktion an: Eine langsame, sanfte, stetige Dehnung ist die Voraussetzung, dass ein Muskel sich öffnet, sonst verkrampft er und lässt sich nur unter sehr unangenehmen Qualen lösen. Die schmerzfreie Öffnung des Schließmuskels ist Voraussetzung dafür, dass der Spülkörper einsetzbar ist. Dazu können folgende Maßnahmen vorgesehen sein: Entkrampfung mit einem Nitropräparat, dem Gleitmittel, eichel- oder geschossartige Form des Spülkörpers und/oder ein steiler Abfall. Bei einer eichel- oder geschossartige Form des Spülkörpers dehnt sich der Schließmuskel beim Einführen des Spülkörpers mit seinem darmseitigen Ende langsam und stetig auf. Sobald der Scheitelpunkt überwunden ist fällt die Form steil ab. Dadurch wird eine sichere Verschlussfunktion erreicht. Durch den steilen Abfall des Segmentes am Ende legt sich der Muskelring optimal an die Segmentwand an.

Die Form des Spülkörpers ist vorzugsweise derart ausgewählt, dass der Muskel den Spülkörper während der Spülung hält. Das erleichtert die Handhabung. Der Nutzer hat die Hände frei, wenn der Spülkörper bis zur passenden Weite eingeführt ist. Zur Entfernung wird der Spülkörper leicht gekippt um ihn zu lösen. Die schonende Aufdehnung wird besonders gut durch eine eichel- bzw. geschossförmige Gestaltung der Einzelsegmente erreicht. Jeder Einzelcorpus weist vorzugsweise einen parabelförmigen Schnitt auf. Da mit der Größe der möglichen Öffnungen der Schließmuskeln auch der Querschnitt dieser beiden Verschlussmuskeln zunimmt, nehmen die Abstände der Einzelsegmente vorzugsweise proportional zum Durchmesser zu. Dadurch passen sie sich optimal und schmerzfrei ein.

Vorzugsweise ist ein Stopper vor dem in den Darm einzuführenden Teil der Darmrohr-Anordnung angeordnet. Mit dem Stopper wird verhindert, dass der Spülkopf-Aufsatz zu weit in den Darm eingeführt wird und möglicherweise Verletzungen hervorruft. Der Stopper kann von einer konisch zulaufenden Manschette oder einem Rand gebildet sein. Vorzugsweise ist der Stopper an den letzten Teilabschnitt des in den Darm einzuführenden Teils der Darmrohr-Anordnung bzw. an den Spülkörper-Aufsatz angeformt.

In einer weiteren Ausgestaltung der Erfindung ist die Wasserversorgung von einem Reservoir mit begrenztem Volumen gebildet oder es ist ein Reservoir mit begrenztem Volumen zwischen der Wasserversorgung und dem Griffteil vorgesehen. Ein solches Reservoir kann beispielsweise von einer Blasenspritze gebildet sein. Das Reservoir sollte bei Darmrohr-Anordnungen zur Darmspülung von Erwachsenen ein Volumen von 200 ml nicht übersteigen. Mit dem Reservoir wird verhindert, dass zu viel Wasser in den Darm gelangt und Verletzungen vorgebeugt.

Vorzugsweise ist ein an dem Griffteil vorgesehenes Ventil zur Freigabe einer Wasserströmung vorgesehen. Das Griffteil kann auch direkt mit einem Brauseschlauch an eine Wasserversorgung, etwa den Hahn eines Bidets, eines Waschbeckens oder einer sonstigen Badezimmerarmatur angeschlossen werden. Das Ventil erlaubt die individuelle Steuerung der Wassermenge.

Weiterhin kann auf der dem Darm abgewandten Seite des Griffteils ein Luer-Lockanschluss zum Zuführen von Reinigungsmitteln oder anderen Zusätzen, vorzugsweise Glycerin und/oder Elektrolyte vorgesehen sein. Ein Luer-Locksystem ist ein genormtes Verbindungssystem aus dem medizinischen Bereich.

In einer besonders bevorzugten Ausgestaltung der Erfindung ist ein doppelwandiges Schutzkondom mit integriertem Gleitmittelreservoir am darmseitigen Ende der Darmrohr-Anordnung vorgesehen. Dies hat sich als vorteilhafter, als die einfach aufgesetzte Schutzkappe, als Handhabung erwiesen. Mit dem Einführen des Spülkörperaufsatzes tritt das Gleitmittel aus. Bei Kontakt des aufgesetzten Schutzkondoms, wird die im Gleitmittelreservoir enthaltende Salbe entleert. Durch das in der Salbe enthaltende Betäubungsmittel in Kombination mit einer Nitrosalbe wird die empfindliche Analhaut betäubt und die Nitrosalbe entspannt die Schließmuskeln. Damit ist das Einführen des Spülkörperaufsatzes erleichtert. Dies ist besonders bei Patienten, die aufgrund von Hämorrhoiden und Exzementzündungen sehr schmerzempfindlich sind, sinnvoll. Zudem sichert das Gleitmittel den gas- und flüssigkeitsdichten Verschluss der aboralen Öffnung bei eingeführtem Spülkörper.

Das Schutzkondom mit integriertem Gleitmittelreservoir ist vorzugsweise doppelwandig ausgebildet. Es besteht aus einer kräftigeren, d.h. weniger zerreißenden Rückwand und einer dünneren Vorderwand, falls erwünscht mit einem aufgesetzten Zentrierkörper, aus einem glukosehaltigem oder anderem wasserlöslichem Produkt. Das glukosehaltige Produkt kann sich später im Darm auflösen. Der Zentrierkörper zerreißt beim Aufsetzen des Spülrohres, so dass das Gleitmittel frei wird.

An seiner vorderen Spitze hat das Schutzkondom eine Öffnung, kann aber auch in Abhängigkeit von dem verwendeten Material geschlossen sein, wenn sichergestellt ist, dass sich das Schutzkondom intakt einbringen lässt und dann mit dem Einlauf der Spülflüssigkeit so rasch auflöst, dass der Fluss der Spülflüssigkeit nicht behindert wird. Dass doppelwandige Schutzkondom ist als Einmalprodukt wesentlich kostengünstiger als ein Wechsel des gesamten Spülkörpers im Einmalgebrauch. Ein vollständiger Ersatz des Spülkörpers, wenn er zur reinen Spülung in Verwendung ist, nach jedem Gebrauch ist dann nicht mehr notwendig. Das Schutzkondom kann aus einer wasserlöslichen Folie z.B. Polyvinylalkohol (PVAL) bestehen. Hierbei handelt es sich um eine kaltwasserlösliche Folie, die sich beim Kontakt mit wässrigen Lösungen vollständig auflöst. Sie ist im Übrigen aber gasdicht und beständig gegen viele organische Lösungsmittel, wie Aromate und Aliphate, sowie gegen mineralische und vegetabilische Öle und Fette, so dass sich Salben sicher darin aufbewahren lassen. Diese Schutzkondome werden bis zur Verwendung in kleinen aufreißbaren Behältnissen (z.B. Aludöschen) verwahrt und dann unmittelbar vor Gebrauch entnommen, das Behältnis wird verworfen. Das Schutzkondom ist doppelwandig. Die äußere Hülle ist etwas dünner gestaltet und hat einen höheren Reibungswiderstand z.B. durch aufgesetzte, feine Wälle o.ä. und hat an den kritischen Punkten (Spitze und über den Segmentzwischenräumen) kleine Sollbruchlinien. Ein Wiedergebrauch ist nicht möglich, da es durch die Nutzung zerstört wird. Allfällige Folienreste, die im Darm verbleiben, lösen sich vollständig auf.

Mit der beschriebenen Darmrohr-Anordnung können die eingangs beschriebenen Probleme weitgehend gelöst werden. Die insbesondere altersbedingte, verlangsamte Nahrungs- und Stuhlpassage durch den Darm kann mit der Darmspülung unterstützt werden. Kotsteinbildung wird vermieden.

Die Erfindung wird im Folgenden anhand von Ausführungsbeispielen und Ausführungsvarianten unter Bezugnahme auf die beigefügten Figuren näher erläutert, wobei die Erfindung hierdurch nicht eingeschränkt wird. Dabei sind die Merkmale der verschiedenen Ausführungsbeispiele und Ausführungsvarianten sowohl einzeln als auch in einer beliebigen Kombination miteinander vorteilhaft.

### Figuren

- Figur 1: zeigt schematisch eine perspektivische, teilgeschnittene Ansicht eines Darmspülrohres mit Spülkörper-Aufsatz.
- Figur 2: zeigt schematisch das Darmspülrohr aus Figur 1 im Querschnitt.
- Figur 3: zeigt schematisch einen Querschnitt durch den in Figur 2 mit 1 bezeichneten Bereich.
- Figur 4: zeigt schematisch einen Querschnitt durch den in Figur 2 mit 2 bezeichneten Bereich.
- Figur 5: zeigt schematisch einen Querschnitt durch den in Figur 2 mit 3 bezeichneten Bereich.
- Figur 6: zeigt schematisch eine teilgeschnittene, perspektivische Ansicht des Endsegments des Spülkörper-Aufsatzes.
- Figur 7: zeigt schematisch eine teilgeschnittene, perspektivische Ansicht des Zwischensegments des Spülkörper-Aufsatzes.
- Figur 8: zeigt schematisch eine teilgeschnittene, perspektivische Ansicht des Eingangssegments des Spülkörper-Aufsatzes.
- Figur 9: zeigt schematisch ein Querschnitt durch einen Zufuhrschlauch aus Silikon.
- Figur 10: zeigt schematisch ein Querschnitt durch das Endsegment aus Figur 6.
- Figur 11: zeigt schematisch die Wasserversorgung für das Darmspülrohr aus Figur 1.
- Figur 12: zeigt schematisch ein Längsschnitt in dem mit 6 bezeichneten Bereich im Detail in Figur 11.
- Figur 13: zeigt schematisch ein Querschnitt durch den mit 4 bezeichneten Bereich in Figur 11.
- Figur 14: zeigt schematisch eine separate Darstellung eines Gleitmittelreservoirs zum Aufsetzen auf die Spitze des Spülkörper-Aufsatzes.
- Figur 15: zeigt schematisch einen Längsschnitt durch einen Teil einer erfindungsgemäßen Darmrohr-Anordnung.
- Figur 16: zeigt schematisch einen Längsschnitt durch einen Teil eines erfindungsgemäßen Spülkörpers.
- Figur 17: zeigt schematisch eine perspektivische, teilgeschnittene Ansicht der Darmrohranordnung mit Griff und Spülkörper-Aufsatz.
- Figur 18: zeigt schematisch eine Aufsicht auf den Spülkörperaufsatz aus Vogelperspektive zur Darstellung der Linkskrümmung.
- Figur 19: zeigt schematisch die Darmrohranordnung aus Figur 17 im Längsschnitt.
- Figur 20: zeigt schematisch einen Längsschnitt durch den Griff mit Steck- und Muffenanschlüssen unter dem Steuerventil.
- Figur 21: zeigt schematisch einen Längsschnitt durch den schaumproduzierenden Griff mit Steck- und Muffenanschlüssen unter dem integrierten Steuer-/Rückschlagventil.
- Figur 22: zeigt schematisch ein schaumproduzierendes Detail aus dem Griffteil und entspricht Patent Nr. DE 3328530 A1 vom 06.08.1983.
- Figur 23: zeigt schematisch als Detail den Luer-Lock-Anschluss im Griffteil.
- Figur 24: zeigt schematisch als Detail den Luer-Lock-Anschluss im schaumproduzierenden Griffteil.
- Figur 25: zeigt schematisch eine Detaildarstellung des integrierten Steuer-/ Rückschlagventils, sowie einen Querschnitt in Höhe des Ventilkopfes.
- Figur 26: zeigt schematisch eine teilgeschnittene, perspektivische Ansicht des Spülkörper-Aufsatzes mit Stopper.
- Figur 27: zeigt schematisch eine Darstellung der Einzelsegmente des Spülkörperaufsatzes in der Reihenfolge Endsegment, Zwischensegment und Eingangssegment.
- Figur 28: zeigt schematisch ein Querschnitt des Griffs in Höhe des Luer-Lock-Anschlusses.
- Figur 29: zeigt schematisch einen Querschnitt des schaumproduzierenden Griffs in Höhe des Luer- Lock-Anschlusses.
- Figur 30: zeigt schematisch einen Querschnitt des Griffs.
- Figur 31: zeigt schematisch einen Querschnitt des schaumproduzierenden Griffs.
- Figur 32: zeigt schematisch einen Querschnitt des Spülkörpers in Höhe des Endsegmentes.
- Figur 33: zeigt schematisch einen Querschnitt des Spülkörpers in Höhe des Zwischensegmentes.
- Figur 34: zeigt schematisch einen Querschnitt des Spülkörpers in Höhe des Eingangssegmentes.
- Figur 35: zeigt schematisch eine perspektivische Ansicht des Spülkörperaufsatzes mit aufgesetztem Schutzkondom.
- Figur 36: zeigt schematisch einen Längsschnitt des Spülkörperaufsatzes mit einem doppelwandigen Schutzkondom mit einem Gleitmittelreservoir.
- Figur 37: zeigt schematisch eine perspektivische Ansicht des Schutzkondoms mit integrierter Öffnung.
- Figur 38: zeigt schematisch ein Querschnitt in Höhe zu in Fig. 41 eingezeichneter Schnittebene.
- Figur 39: zeigt schematisch eine perspektivische Ansicht durch eine Schlauchverbindung für die Flüssigkeitszufuhr.
- Figur 40: zeigt schematisch eine perspektivische Ansicht des Steckanschlusses und ein Längsschnitt durch die Flüssigkeitssteckdose.
- Figur 41: zeigt schematisch einen Längsschnitt und eine Seitenansicht der Wassersteckdose.
- Figur 42: zeigt schematisch eine perspektivische Ansicht eines Flüssigkeitsboilers mit Mischbatterie.
- Figur 43: zeigt schematisch eine perspektivische Ansicht und einen Querschnitt der Flüssigkeitszufuhr durch eine Toilettenbrille.
- Figur 44: zeigt schematisch eine perspektivische Ansicht und einen Querschnitt der Flüssigkeitszufuhr durch den Oberrand einer Toilettenschüssel.
- Figur 45: zeigt schematisch eine Gesamtdarstellung der Fig. 1 in Kombination mit Fig. 42-44.
- Figur 46: zeigt schematisch ein Zusatzelement einer Toilettenbürste mit Warmflüssigkeitszufuhr.
- Figur 47: zeigt schematisch die Wasserversorgung für das Darmspülrohr aus Figur 1.

### Beschreibung der Ausführungsbeispiele

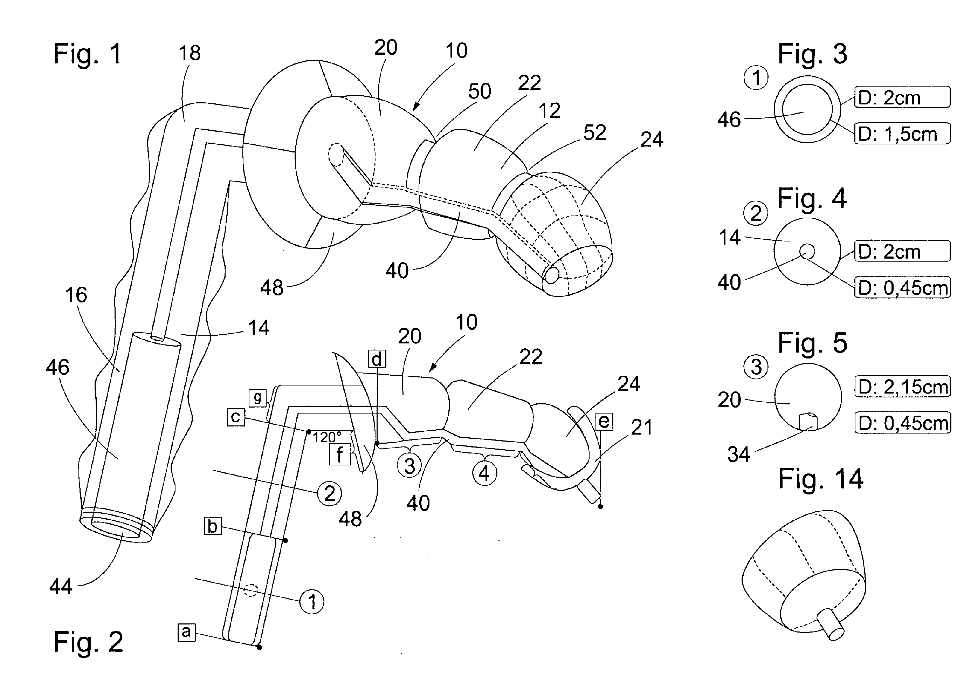

Ein erstes Ausführungsbeispiel wird im Folgenden anhand der Figuren 1 bis 14 und 35 bis 38 näher erläutert.

Figuren 1 und 2 zeigen ein allgemein mit 10 bezeichnetes Darmrohr. Das Darmrohr 10 weist darmseitig einen Spülkopf-Aufsatz 12 und ein Griffteil 14 auf. Das Griffteil 14 ist mit einem Handgriff versehen. Griffteil 14 und Spülkopf-Aufsatz 12 sind mit Silikon beschichtet. Das erleichtert die Reinigung und Handhabung. Der Spülkopf-Aufsatz 12 ist auf das Griffteil 14 aufsteckbar. Das Griffteil 14 ist im Bereich 18 vor dem Spülkopf-Aufsatz 12 um etwa 70° gewinkelt.

Der aufgesteckte Spülkopf-Aufsatz 12 ist im vorliegenden Ausführungsbeispiel als Einwegteil ausgebildet und wird nach der Anwendung entsorgt. In einem nicht dargestellten Ausführungsbeispiel ist der Spülkopf-Aufsatz 12 aus einem leicht zu reinigenden und desinfizierenden Material ausgebildet.

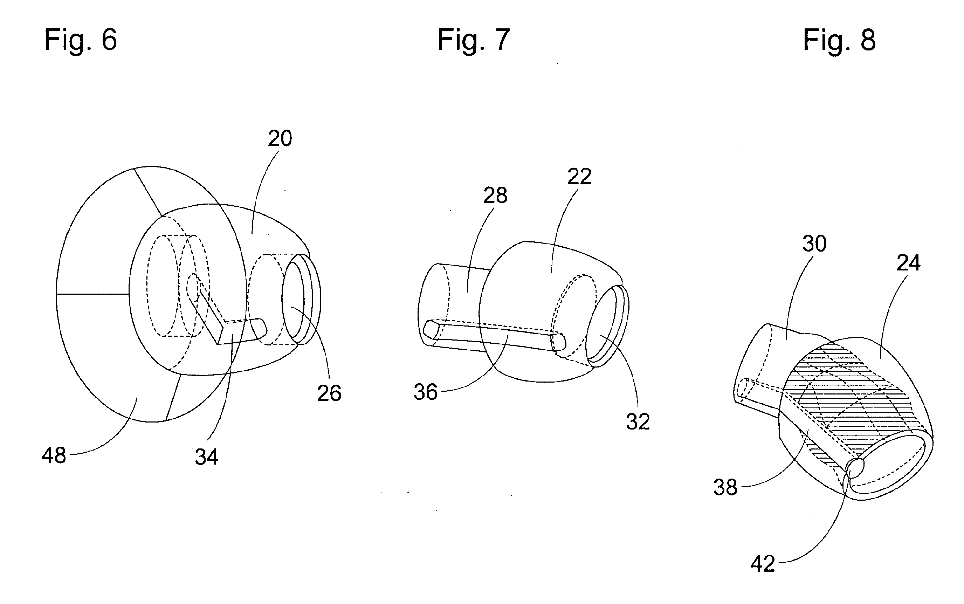

Der Spülkopf-Aufsatz 12 besteht zur erleichterten Fertigung aus drei Abschnitten 20, 22 und 24, die in den Figuren 6 bis 8 gesondert dargestellt sind. Der Abschnitt 20 ist ein Endsegment. Der Abschnitt 22 ist ein Zwischensegment. Der Abschnitt 24 ist ein Eingangssegment. Das Endsegment ist darmseitig mit einer Verjüngung 26 versehen (Figur 6). Das Zwischensegment weist einen korrespondierenden Vorsprung 28 auf. Die Länge des Vorsprungs 28 bestimmt den Abstand der Strecke zwischen den Segmenten und ist proportional zum Durchmesser des Segments. Das Eingangssegment weist einen Vorsprung 30 auf. Das Zwischensegment weist eine korrespondierende Verjüngung 32 auf. Die Vorsprünge 30 und 28 werden in die korrespondierenden Verjüngungen 26 und 32 gesteckt. Jeder der Abschnitte 20, 22 und 24 weist im Inneren einen Kanalabschnitt 34, 36 und 38 auf. Die Abschnitte 20, 22 und 24 werden derart zusammengesteckt, dass die Kanalabschnitte 34, 36 und 38 einen Kanal 40 bilden, wie dies in den Figuren 1 und 2 dargestellt ist.

Das Zwischensegment 22 und das Eingangssegment 24 sind gekrümmt ausgebildet. Dadurch ergibt sich im zusammengesetzten Zustand ein gekrümmter Spülkörper-Aufsatz 12, wie dies in Figuren 1 und 2 dargestellt ist. Der Kanal 40 verläuft auf der der (gedachten) Krümmungsachse zugewandten Innenseite der Abschnitte 20, 22 und 24. Entsprechend liegt die Mündung 42 des Kanals 40 am darmseitigen Rand des Eingangssegments 24. Dadurch wird eine wandnahe Spülung erreicht.

Auf der dem Darm abgewandten Seite 44 des Griffteils 14 ist der Kanal 40 in einem Bereich 46 verbreitert. Dies ist auch in den Querschnitten in den Figuren 3 bis 5 zu erkennen. In dem in Figur 2 mit "2" markierten Bereich verläuft der Kanal 40 mit geringem Durchmesser mittig durch das Griffteil 14. Dies ist in Figur 4 dargestellt. In den in Figur 2 mit "3", "4" und "5" markierten Bereichen 34 verläuft der Kanalabschnitt 34 im Bereich des Randes des Endsegments 20 bzw. des Zwischensegments 22 und des Eingangssegments 24. Dies ist in den Figuren 5 und 50 dargestellt. In dem in Figur 2 mit "1" markierten Bereich 46 ist der Kanal 40 verbreitert um das Darmrohr 10 an eine nachstehend beschriebene Wasserversorgung anschließen zu können. Dies ist in Figur 3 dargestellt.

Das Endsegment 20 weist einen Stopper 48 auf. Der Stopper 48 besteht aus einem konisch zulaufenden, an das Endsegment 20 angeformten, vorspringenden Ring. Der Stopper 48 begrenzt die Einführungstiefe des Darmrohrs. Dadurch werden Verletzungen bei der Anwendung sicher vermieden. Nur die drei Abschnitte 20, 22 und 24 des Spülkopf-Aufsatzes 12 können in den Darm eingeführt werden.

Beispielhaft sind die Abmessungen für den Spülkopf-Aufsatz 12 für einen Erwachsenen angegeben (die Bereichsangaben beziehen sich auf Figur 2):

| Längen | Durchmesser |
|---|---|
| von a bis b = 60 mm | Bereich 3: Anfang 17 mm |
| von a bis c = 100 mm | Bereich 4: Anfang 16 mm, Ende 14,5 mm |
| von c bis d = 20 mm | Bereich 5: Anfang 14,5 mm, Ende 12 mm |
| von d bis e = 86 mm | |
| f = 20 mm, Winkel ca. 60° | |
| g = 20 mm | |

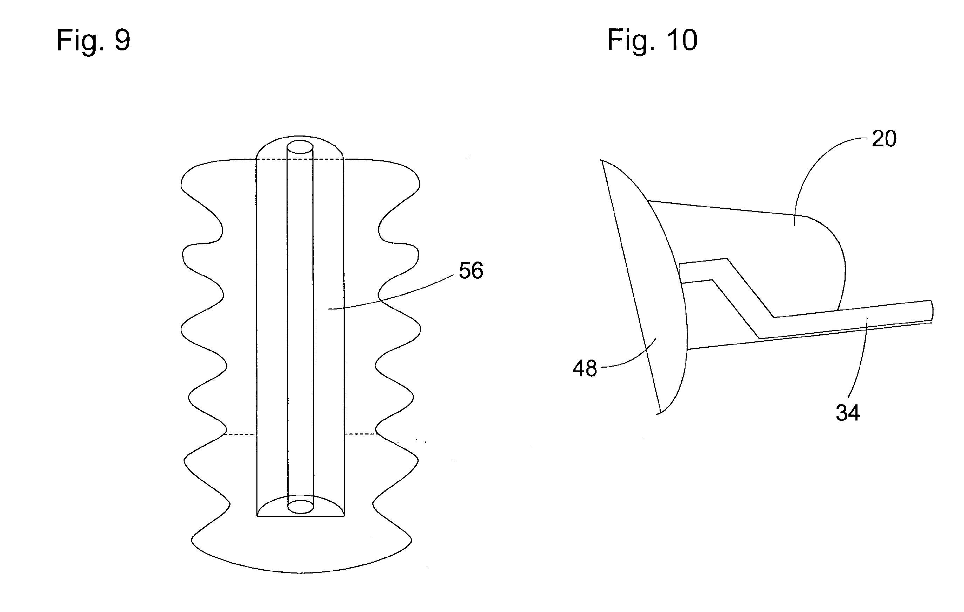

Man erkennt, dass der Stopper 48 einen ca. 40 mm größeren Durchmesser hat und daher praktisch nicht einführbar ist. Figur 10 zeigt das Endsegment noch einmal im Detail. Die oben aufgeführten Abmessungen können individuell an die Bedürfnisse des Nutzers angepasst werden. So sind Durchmesser im Bereich zwischen 8 und 20 mm sinnvoll. Eine parabelförmige Wandung, die der natürlichen spastischen Verschlussreaktion der Schließmuskular angepasst ist, erleichtert die Einführung.

Zwischen den zusammengesetzten Abschnitten 20, 22 und 24 sind im Bereich der Verbindungsstellen Verjüngungen 50 und 52 gebildet. Diese erlauben einen Verschluss während des Einführens, so dass der Nutzer sich nicht beschmutzt.

Auf die Spitze des Eingangssegments des Spülkörper-Aufsatzes ist ein Gleitmittelreservoir 21 aufgesetzt. Das Gleitmittelreservoir 21 ist im vorliegenden Ausführungsbeispiel doppelwandig ausgebildet. Es ist in Figur 14 noch einmal separat dargestellt. Es besteht aus einer kräftigeren, d.h. weniger zerreißenden Rückwand und einer dünneren Vorderwand mit einem aufgesetzten Zentrierkörper aus einem glukosehaltigem Produkt. Das glukosehaltige Produkt kann sich später im Darm auflösen. Der Zentrierkörper zerreißt beim Aufsetzen des Darmrohres, so dass das Gleitmittel frei wird.

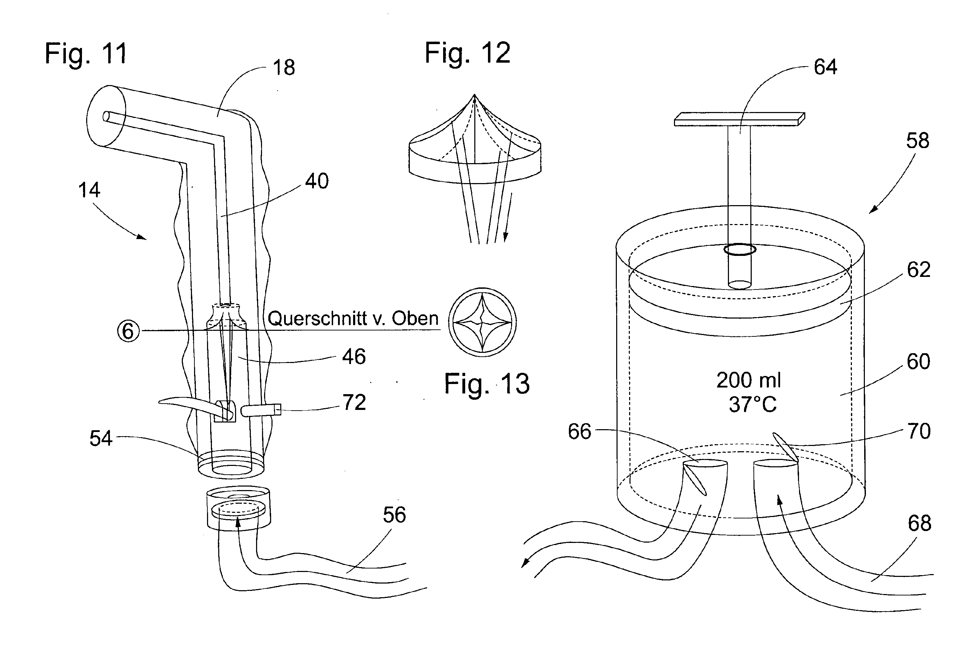

Figur 11 bis 13 illustrieren die Wasserversorgung des Darmrohrs 10. Das Griffteil 14 ist mit einem Halbzoll-Außengewinde 54 versehen. An das Gewinde 54 ist ein in Figur 9 im Detail dargestellter Zufuhrsilikonschlauch 56 angeschlossen. Der Zufuhrsilikonschlauch 56 wird mit Wasser aus einem Wasserreservoir 58 gespeist. Im vorliegenden Ausführungsbeispiel ist das Wasserreservoir 58 von einem wärmeisolierten 200 ml- Zylinder 60 mit einem Kolben 62 gebildet. Der Kolben 62 ist mit einem Griff 64 versehen.

Wenn der Kolben 62 mit dem Griff 64 nach unten bewegt wird, wird Wasser aus dem Zylinder durch den Zufuhrsilikonschlauch 56 in das Darmrohr geleitet. Zur Vermeidung von Verunreinigungen in dem Zylinder 60 ist ein Rückflussverhinderer 66 am Eingang des Zufuhrsilikonschlauches 56 vorgesehen. Das Wasser kann also nur in Richtung des Darmrohres 10 fließen.

Zum Befüllen des Zylinders 60 ist ein Wasseranschluss über einen Schlauch 68 vorgesehen. Der Schlauch 68 kann an jede beliebige Zapfstelle, etwa einen Wasserhahn angeschlossen werden. Auch dieser Schlauch ist mit einem Rückflussverhinderer 70 im Bereich des Zylinders 60 versehen. Mit dem zwischen Wasserhahn und Darmrohr vorgesehenen Zylinder wird das maximale Spülvolumen definiert und Verletzungen durch zu große Mengen Spülflüssigkeit verhindert.

Im vorliegenden Ausführungsbeispiel wird der Schlauch 68 an eine Warmwasserversorgung angeschlossen und das Wasser auf 37°C temperiert eingeführt. In einem - nicht dargestellten - alternativen Ausführungsbeispiel umfasst der Zylinder einen Thermostat, mit einer Heizung, so dass kaltes Wasser auf die gewünschte Temperatur geheizt werden kann.

Im Bereich 46 des Griffteils 14 ist ferner ein Luer-Lock Anschluss 72 vorgesehen. Über diesen Anschluss 72 können der Spülflüssigkeit Reinigungsmittel, etwa Glycerin oder Elektrolyte zugeführt werden.

Das beschriebene Darmrohr 10 arbeitet wie folgt: Zunächst wird ein neuer, zusammengesteckter Spülkopf-Aufsatz 12 auf das Griffteil 14 aufgesteckt. Anschließend werden ca. 200 ml warmes Wasser durch Öffnen des Wasserhahns in den Zylinder 60 eingefüllt. Dann wird der Wasserhahn wieder geschlossen. Das Darmrohr 10 kann nun mit dem Spülkopf-Aufsatz 12 schrittweise in den Darm eingeführt werden. Der Nutzer hält den Handgriff 16 vorne und führt das Darmrohr mit dem darmseitigen Eingangssegment 24 schräg oben nach ein. Wenn das Darmrohr 10 bis zum Stopper 48 eingeführt ist, wird der Kolben 62 mit dem Griff 64 nach unten bewegt. Wasser fließt durch das Darmrohr 10 in den Darm. Während der Spülung kann das Darmrohr 10 leicht gedreht werden. Die Spülflüssigkeit fließt dann durch die außeraxiale Lage des Kanals 40 kreisförmig entlang der Darminnenwand.

Patienten mit Schwierigkeiten bei der Handhabung setzen eine Kappe (nicht dargestellt) auf das Eingangssegment 24. Die Kappe enthält eine Salbe, die neben guten Gleiteigenschaften auch ein lokal anzuwendendes Betäubungsmittel aufweist. Die Kappe gibt die Salbe bei der Einführung frei und erleichtert die Handhabung. Das Betäubungsmittel führt zu einer Vermeidung von Schmerzen und zur Entspannung des Schließmuskels.

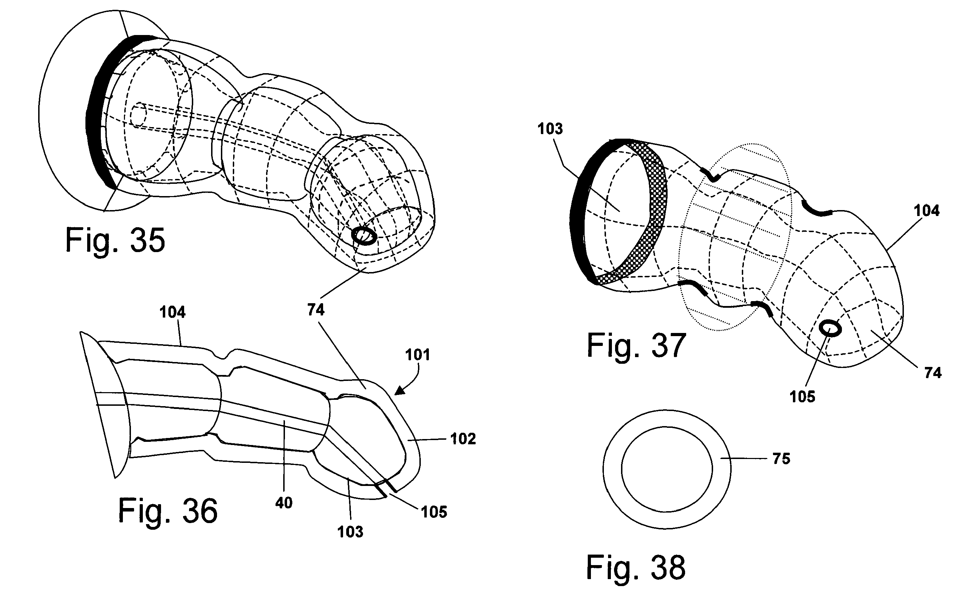

Ein Gleitmittelreservoir-Aufsatz bzw. eine zuvor beschriebene Kappe 100 zum Aufsetzen auf die Spitze des Spülkörpers ist schematisch in der Figur 14 gezeigt. Alternativ dazu kann der Aufsatz mit dem Gleitmittelreservoirs als doppelwandiges Schutzkondom 101 mit einem Gleitmittelreservoir 102 ausgestaltet sein. Die Figur 36 zeigt schematisch einen Längsschnitt des Spülkörperaufsatzes mit einem doppelwandigen Schutzkondom mit einem Gleitmittelreservoir. Das Schutzkondom umfasst eine innere Wand oder Hülle 103 und eine äußere Wand oder Hülle 104 zwischen denen ein Reservoir 102 ausgebildet ist. Die Figur 37 zeigt schematisch eine perspektivische Ansicht des Schutzkondoms mit integrierter Öffnung. Das Schutzkondom umfasst eine Öffnung 105, die entsprechend der Position der Austrittsöffnung des Fluidkanals 40 angeordnet ist.

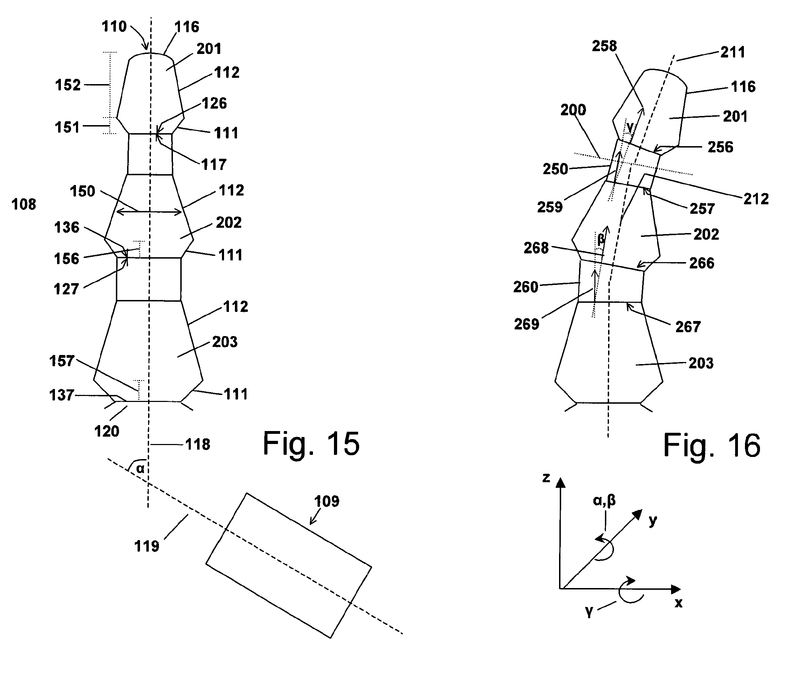

Eine zweite Ausführungsvariante wird im Folgenden unter Bezugnahme auf die Figuren 15 und 16 näher erläutert. Die Figur 15 zeigt schematisch einen Längsschnitt eines Teils einer erfindungsgemäßen Darmrohr-Anordnung. Die Darmrohr-Anordnung umfasst ein Griffteil 109, welches in der Figur 15 nur in Form eines Teilbereiches gezeigt ist, und einen Spülkörper 108. Das Griffteil 109 umfasst eine Mittelachse 119.

Der Spülkörper umfasst ein distales Ende 110 und ein proximales Ende 120. Weiterhin umfasst der Spülkörper eine Anzahl Segmente 201, 202, 203. Jedes Segment weist eine Mittelachse 118 auf. In der Figur 15 sind die Mittelachsen der Segmente 201, 202, 203 identisch und bilden gleichzeitig die Mittelachse 118 des Spülkörpers 108. Für den Fall, dass die Lage der Mittelachsen der Segmente voneinander abweichen, ist die Mittelachse des Spülkörpers 108 durch die Mittelachse des am proximalen Ende 120 des Spülkörpers 108 angeordneten Segments, in der Figur 15 des Segments 203, definiert.

Die Mittelachse 119 des Griffteils und die Mittelachse 118 des Spülkörpers sind schließen einen Winkel α, der zwischen 50° und 80°, vorzugsweise zwischen 65° und 75°, beträgt. In der Figur 15 sind die Mittelachse 118 des Spülkörpers 108 und die Mittelachse 119 des Griffteils 109 in der x-y-Ebene des in der Figur 15 schematisch gezeigten Koordinatensystems angeordnet. Der Winkel α bewirkt also eine Drehung der Mittelachsen 118 bzw. 119 zueinander um die y-Achse.

Die Segmente 201, 202 und 203 des Spülkörpers 108 können als separate Bauteile ausgestaltet sein, die fest oder lösbar miteinander verbunden sind. Alternativ dazu kann der gesamte Spülkörper 108 aus einem Stück gefertigt sein.

Die Segmente 201, 202 und 203 umfassen jeweils einen Durchmesser 150 in Bezug auf die jeweilige Mittelachse des Segments, einen ersten Bereich 111 und einen zweiten Bereich 112. Das erste Segment 201 umfasst zudem eine Grundfläche 117 und eine Deckfläche 116. Dabei ist die Deckfläche 116 des ersten Segments 201 konvex, also nach außen, gekrümmt bzw. gewölbt ausgestaltet, um ein Einführen in den Darm zu erleichtern. Das zweite Segment 202 umfasst eine Grundfläche 127 und eine Deckfläche 126 und das dritte Segment 203 umfasst eine Grundfläche 137 und eine Deckfläche 136. Der erste Bereich 111 schließt sich jeweils an die Grundfläche an, gefolgt von dem zweiten Bereich, der sich seinerseits an die Deckfläche anschließt. Der erste Bereich ist also jeweils zwischen der Grundfläche und den zweiten Bereich angeordnet und der zweite Bereich ist jeweils zwischen der Deckfläche und dem ersten Bereich angeordnet. Grundsätzlich bildet die Grundfläche 117, 127, 137 das proximale Ende und die Deckfläche 116, 126, 136 das distale Ende des jeweiligen Segments 201, 202, 203.

Der Durchmesser des ersten Bereiches 111 nimmt jeweils von der Grundfläche 117, 127, 137 zur Deckfläche 116, 126, 136 hin stetig zu, beispielsweise linear oder kontinuierlich. Der Durchmesser des zweiten Bereiches 112 nimmt jeweils von der Grundfläche 117, 127, 137 zur Deckfläche 116, 126, 136 hin stetig ab, beispielsweise linear oder kontinuierlich. Zudem ist die Länge 151 des jeweils ersten Bereiches 111 entlang der jeweiligen Mittelachse 118 des Segments kleiner als die Länge 152 des jeweils zweiten Bereiches 112 entlang der jeweiligen Mittelachse 118 des Segments, vorteilhafterweise kleiner als mindestens ein Drittel der Länge 152 des jeweils zweiten Bereiches 112.

Weiterhin weist jede Deckfläche eine Flächennormale auf, die in Bezug auf das jeweilige Segment nach außen gerichtet ist. Weiterhin weist jede Grundfläche eine Flächennormale auf, die in Bezug auf das jeweilige Segment nach innen gerichtet ist. Dies ist in der Figur 15 beispielhaft für das dritte Segment 203 gezeigt, wobei die Flächennormale 156 der Deckfläche 136 und die Flächennormale 157 der Grundfläche 157 parallel zueinander verlaufen. Abweichend von der üblichen Konvention, nach welcher die Flächennormale einer Oberfläche nach außen gerichtet ist, wird also im Rahmen der vorliegenden Erfindung die Flächennormale der Grundfläche als nach innen gerichtet definiert. Die Flächennormale der Deckfläche wird dagegen im Rahmen der vorliegenden Erfindung konventionell als nach außen gerichtet definiert.

Durch eine unterschiedliche Ausgestaltung der einzelnen Segmente können den jeweiligen anatomischen Gegebenheiten angepasste Knicke oder Krümmungen des Spürkörpers realisiert werden. Hierzu kann beispielsweise die Flächennormale 157 der Grundfläche 137 eines Segments mit der Flächennormale 156 der Deckfläche 136 des Segments einen Winkel β oder γ einschließen, der zwischen 0° und 80°, beispielsweise zwischen 5° und 45°, vorteilhafterweise zwischen 10° und 30°, liegen kann. Dabei kann der Winkel β eine Drehung der jeweiligen Mittelachsen in der x-z-Ebene, also um die y-Achse, bewirken und der Winkel γ eine Drehung der jeweiligen Mittelachsen in der y-z-Ebene, also um die x-Achse, bewirken.

Vorteilhafterweise schließt die Mittelachse des ersten Segments 201 mit der Mittelachse des zweiten Segments 202 einen Winkel γ ein, um beispielsweise bei humanem Gebrauch den Linksknick des Dickdarmes zu berücksichtigen. Weiterhin schließt vorteilhafterweise die Mittelachse eines im Bereich des proximalen Endes 120 des Spülkörpers 108 angeordneten Segments mit der Mittelachse eines dazu in distaler Richtung benachbart angeordneten Segments einen Winkel β ein. Dadurch kann das Einführen des Spülkörpers 108 in den Darm durch den Nutzer erleichtert werden.

Der maximale Durchmesser 150 der jeweils benachbart zueinander angeordneten Segmente nimmt vom distalen Ende 110 zum proximalen Ende 120 hin zu, wobei sich der maximale Durchmesser 150 einander benachbarter Segmente vorzugsweise um 20% bis 40% unterscheidet. Weiterhin kann der Durchmesser der Grundfläche eines Segments zwischen 5% und 15%, beispielsweise 10% geringer sein als der maximale Durchmesser 150 des jeweiligen Segments.

Die Figur 16 zeigt schematisch eine weitere Variante der Ausgestaltung eines Teils des Spülkörpers 108 in einer teilweise perspektivischen Schnittansicht. Der in der Figur 16 gezeigte Teil des Spülkörpers umfasst Segmente 201, 202 und 203, die dieselben Merkmale und Eigenschaften, wie die im Zusammenhang mit der Figur 15 beschriebenen Segmente haben können. Jeweils zwischen zwei benachbarten Segmenten ist ein Verjüngungsbereich 250, 260 angeordnet. Der jeweilige Verjüngungsbereich 250, 260 kann dabei als separates Bauteil ausgestaltet sein oder als ein Teilbereich eines Segments ausgestaltet sein.

Der zwischen dem ersten Segment 201 und dem zweiten angeordnete Verjüngungsbereich 250 umfasst eine Grundfläche 257, die an der Deckfläche des zweiten Segments 202 angeordnet ist und eine Flächennormale 259 aufweist. Der Verjüngungsbereich 250 umfasst zudem eine Deckfläche 256, die an der Grundfläche des ersten Segments 201 angeordnet ist und eine Flächennormale 258 aufweist. Die Flächennormale 259 der Grundfläche 257 erstreckt sich in der x-z-Ebene und schließt mit der Flächennormale 258 der Deckfläche 256 einen Winkel γ ein, der eine Drehung der Flächennormale 258 gegenüber der Flächennormale 259 um die x-Achse bewirkt. Die Flächennormale der Deckfläche 116 an der Mittelachse 211 bzw. die Mittelachse 211 des ersten Segments 201 zeigt dadurch zumindest teilweise in Richtung der y-Achse.

Der durch den Verjüngungsbereich 250 bewirkte Knick oder die bewirkte Krümmung des Spülkörpers 208 in γ-Richtung ist in der Figur 16 durch eine Linie 200 angedeutet, die eine Änderung der Schnittebene kennzeichnet. In der Figur 16 oberhalb der Linie 200 verläuft der gezeigte Längsschnitt entlang der Mittelachse 211 des ersten Segments, während in der Figur 16 der unterhalb der Linie 200 gezeigte Längsschnitt entlang der Mittelachse 212 des zweiten Segments verläuft.

Zwischen den zweiten Segment 202 und dem dritten Segment 203 ist ein weiterer Verjüngungsbereich 260 angeordnet, der eine Grundfläche 267 mit einer Flächennormale 269 und eine Deckfläche 266 mit einer Flächennormale 268 umfasst. Die Grundfläche 267 des Verjüngungsbereichs 260 ist an der Deckfläche des dritten Segments 203 angeordnet. Die Deckfläche 266 des Verjüngungsbereichs 260 ist an der Grundfläche des ersten Segments 201 angeordnet. Die Flächennormale 269 der Grundfläche 267 schließt mit der Flächennormale 268 der Deckfläche 268 einen Winkel β zwischen 0° und 80°, beispielsweise zwischen 5° und 45°, vorteilhafterweise zwischen 10° und 30°, ein. Dabei liegen die Mittelachsen des zweiten Segments 212 und des dritten Segments in der x-z-Ebene.

Mit Hilfe dieser Ausführungsvariante ist es möglich, einzelne rotationssymmetrische Segmente 201, 202 und 203 zu verwenden, die kostengünstig hergestellt werden können, und die jeweils gewünschte Krümmung zwischen benachbarten Segmenten mit Hilfe der Verjüngungsbereiche, die ebenfalls eine einfache Geometrie aufweisen und kostengünstig hergestellt werden können, zu realisieren.

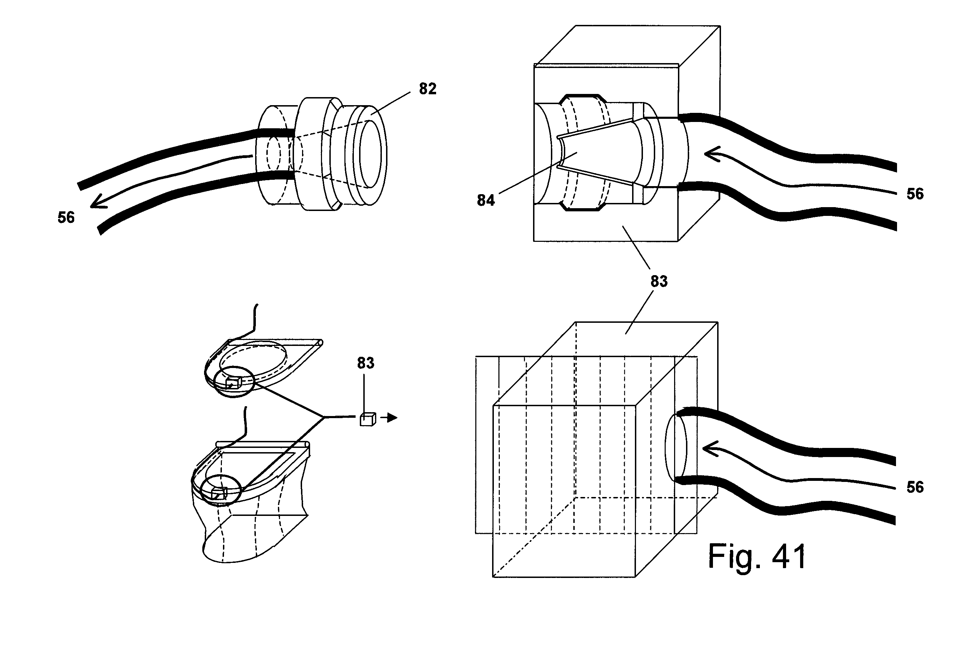

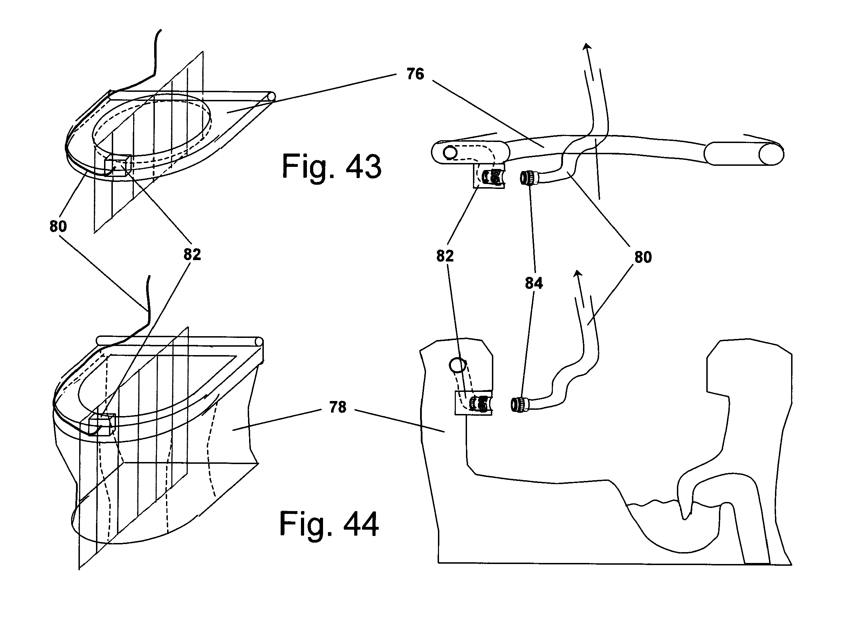

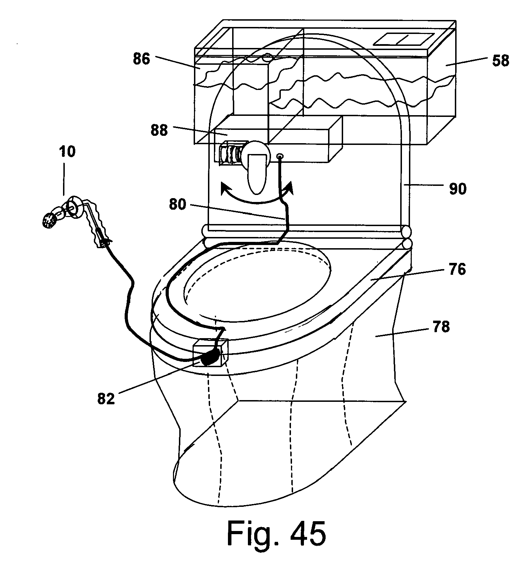

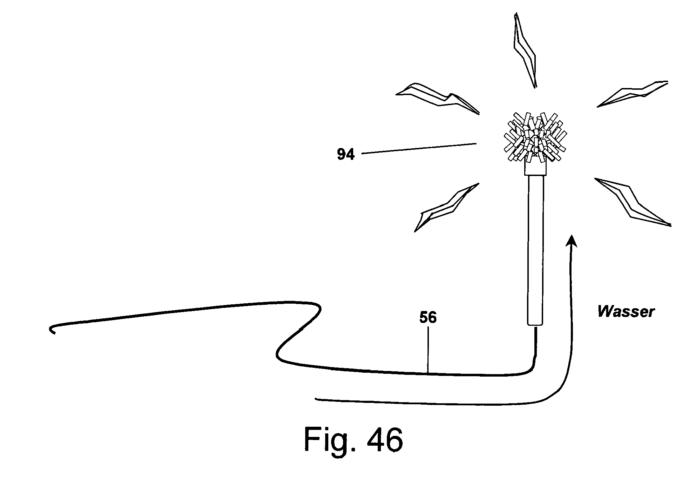

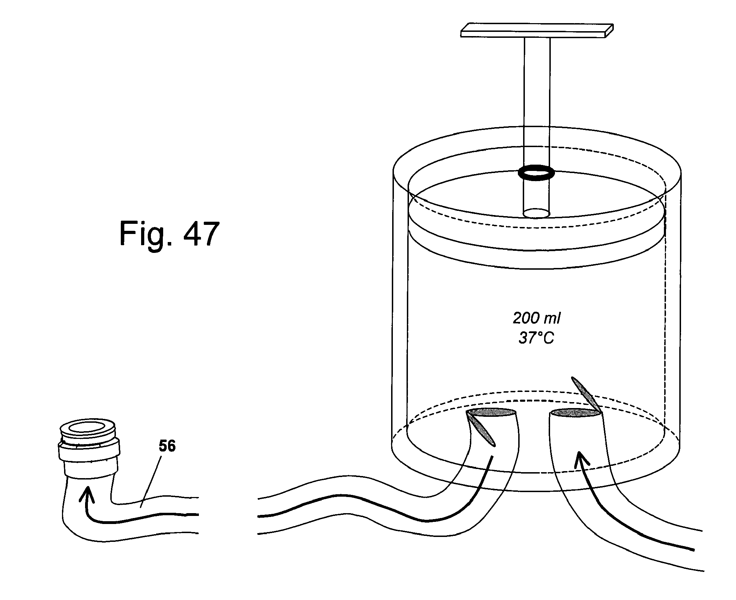

Im Folgenden wird ein weiteres Ausführungsbeispiel anhand der Figuren 1 bis 14, und 17 bis 47 näher erläutert.

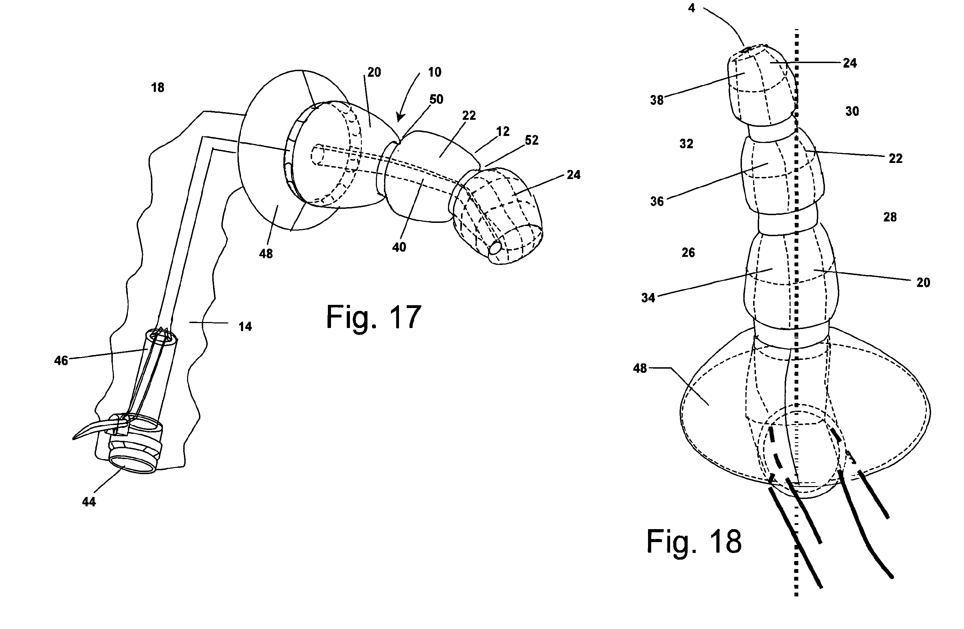

Figur 17 zeigt ein allgemein mit 10 bezeichnetes Darmrohr. Das Darmrohr 10 weist darmseitig einen Spülkopf-Aufsatz 12 und ein Griffteil 14 auf. Das Griffteil 14 ist mit entsprechenden Griffmulden versehen. Griffteil 14 und Spülkopf-Aufsatz 12 sind mit Silikon beschichtet. Das erleichtert die Reinigung und Handhabung. Der Spülkopf-Aufsatz 12 wird mit seinem Muffenanschluss 82 auf das Griffteil 14 über einen Steckanschluss 84 verbunden. Das Griffteil 14 ist im Bereich 18 vor dem Spülkörper-Aufsatz 12 hier als Beispiel um etwa 70° gewinkelt. Der über den Muffenanschluss 82 aufgesteckte Spülkörper-Aufsatz 12 ist im vorliegenden Ausführungsbeispiel als Einwegteil ausgebildet und wird nach der Anwendung entsorgt.

Figur 18 zeigt die Darmrohranordnung aus Vogelperspektive, logischerweise im wesentlichen den Spülkörperaufsatz zur Darstellung der physiologisch wichtigen Linkskrümmung in Ergänzung zur Längskrümmung.

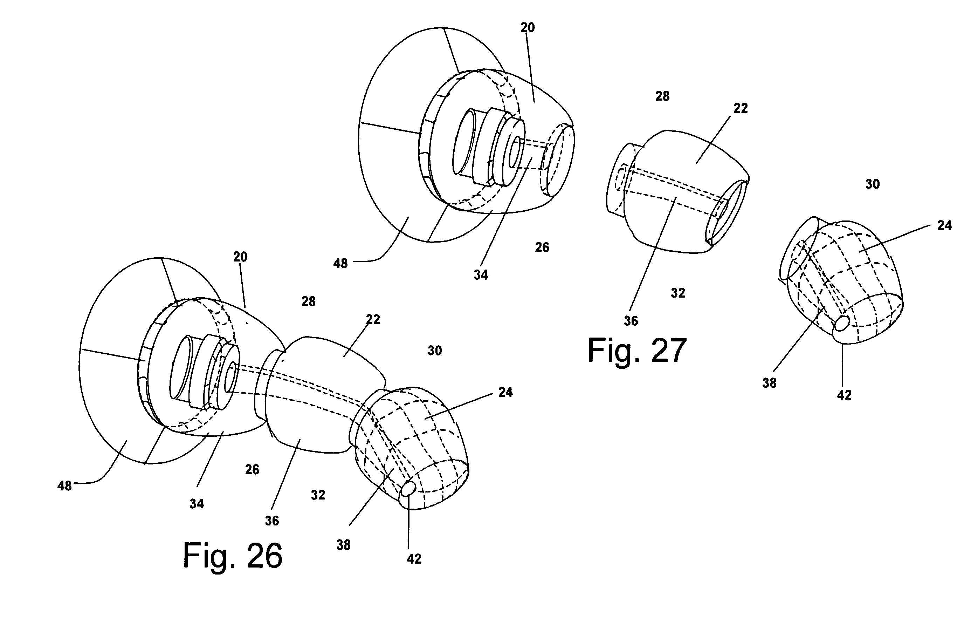

Der Spülkörper-Aufsatz 12 gliedert sich in drei Abschnitten 20, 22 und 24 mit ihrem hyperbolischen Längsschnitt und der sich anschließenden Verjüngung, die in den Fig. 26 und 27 gesondert dargestellt sind. Der Abschnitt 20 ist ein Endsegment. Der Abschnitt 22 ist ein Zwischensegment. Der Abschnitt 24 ist ein Eingangssegment. Das Endsegment ist darmseitig mit einer Verjüngung 26 versehen. Das Zwischensegment weist einen korrespondierenden Vorsprung 28 auf. Die Länge des Vorsprungs 28 bestimmt den Abstand der Strecke zwischen den Segmenten und ist proportional zum Durchmesser des Segments. Das Eingangssegment weist einen Vorsprung 30 auf. Das Zwischensegment weist eine korrespondierende Verjüngung 32 auf. Die Vorsprünge 30 und 28 werden in die korrespondierenden Verjüngungen 26 und 32 gesteckt. Jeder der Abschnitte 20, 22 und 24 weist im Inneren einen Kanalabschnitt 34, 36 und 38 auf. Die Abschnitte 20, 22 und 24 werden derart zusammengesteckt, dass die Kanalabschnitte 34, 36 und 38 einen Kanal 40 bilden, wie dies in der Figur 1 dargestellt ist.

Das Zwischensegment 22 und das Eingangssegment 24 sind gekrümmt ausgebildet. Dadurch ergibt sich im zusammengesetzten Zustand ein ggf. längs- und ggf. nach links gekrümmter Spülkörper-Aufsatz 12, wie dies in Figuren 1 bis 2 dargestellt ist. Der Kanal 40 verläuft auf der der (gedachten) Krümmungsachse zugewandten Innenseite der Abschnitte 20, 22 und 24. Entsprechend liegt die Mündung 42 des Kanals 40 am darmseitigen Rand des Eingangssegments 24. Dadurch wird eine wandnahe Spülung erreicht.

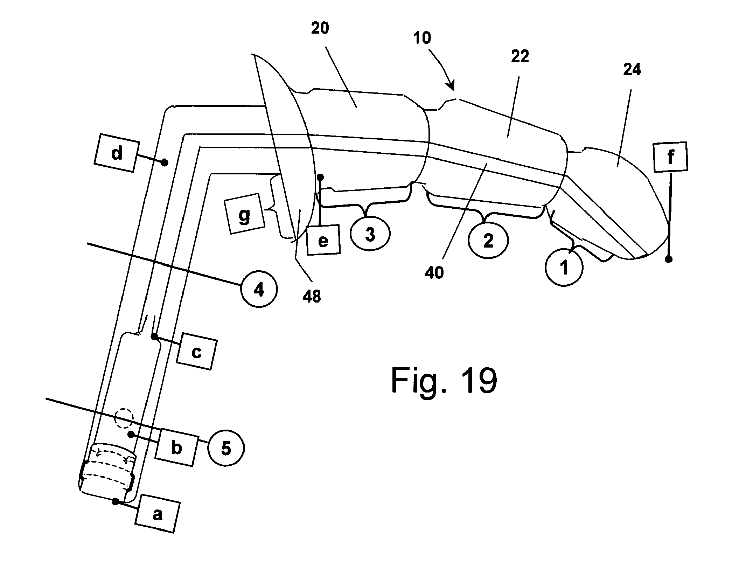

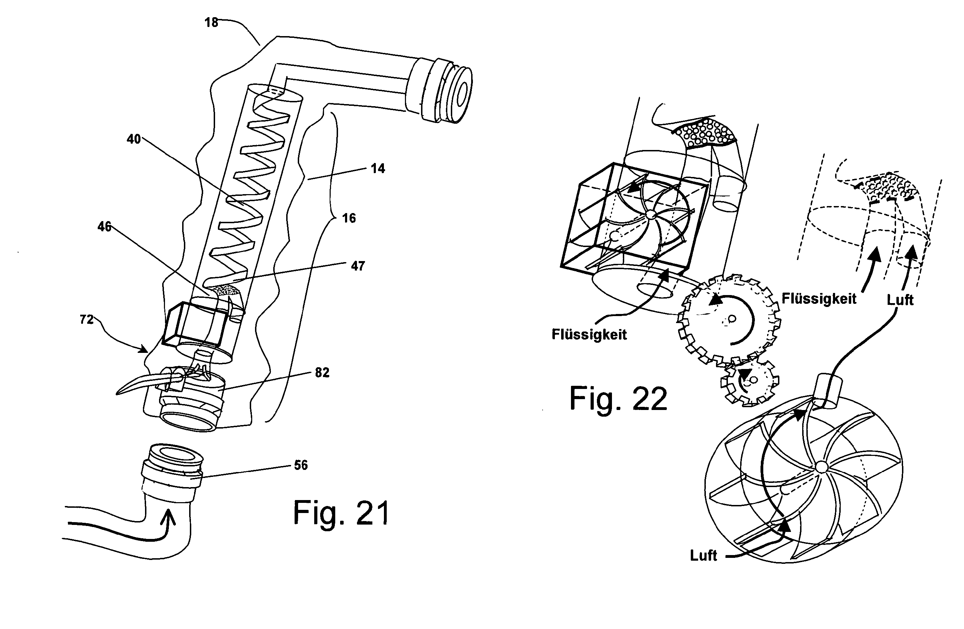

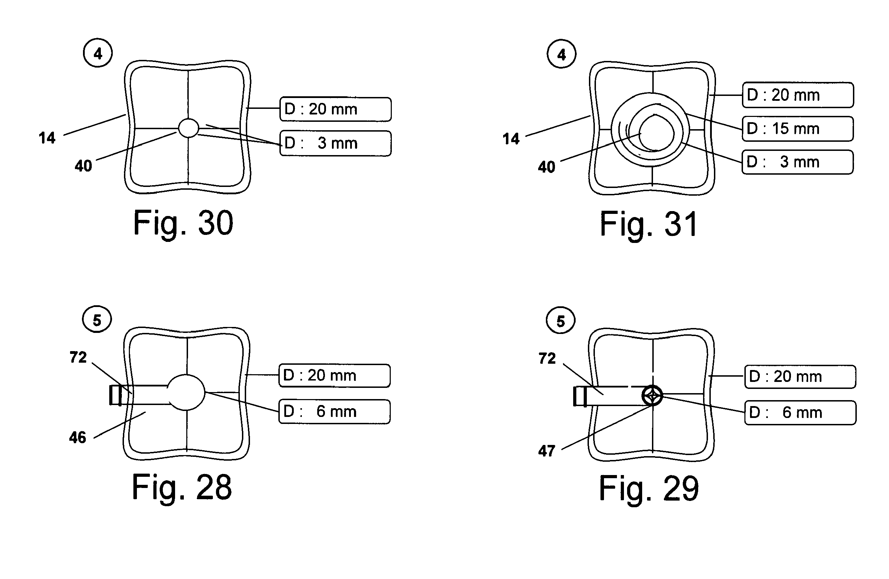

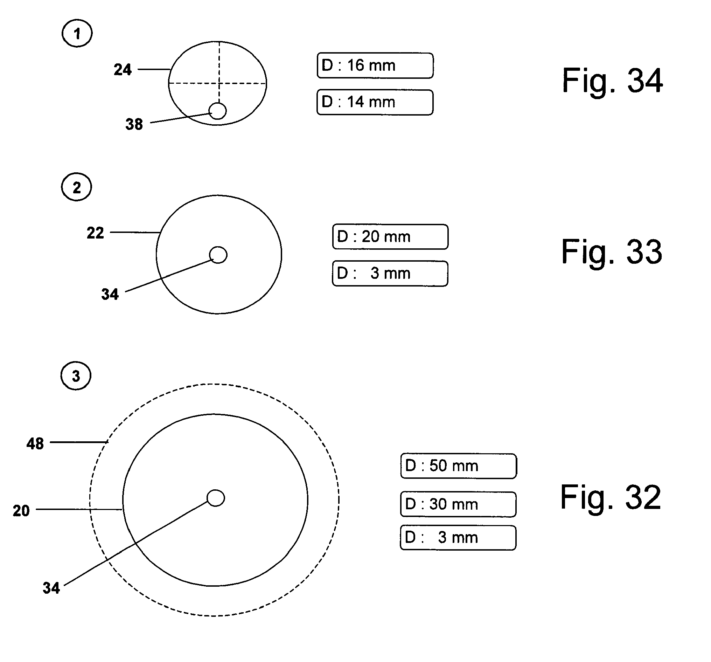

Auf der dem Darm abgewandten Seite 44 des Griffteils 14 ist der Kanal 40 in einem Bereich 46 verbreitert. Dies ist auch in den Querschnitten in den Figuren 28 bis 31 zu erkennen. In dem in Figur 19 mit "4" markierten Bereich verläuft der Kanal 40 mit geringem Durchmesser mittig durch das Griffteil 14. Dies ist in Figuren 30 und 31 dargestellt. In dem in Figur 2 mit "3" markierten Bereich verläuft der Kanalabschnitt 34 zentrisch durch das Endsegments 20. Dies ist in Figur 32 dargestellt. In dem in Figur 2 mit "5" markierten Bereich 46 ist d er Kanal 40 verbreitert um das Darmrohr 10 an eine nachstehend beschriebene Flüssigkeitsversorgung bzw. Schaumapplikator anschließen zu können. Dies ist in Figuren 21 und 22 dargestellt.

Das Endsegment 20 weist einen Stopper 48 auf. Der Stopper 48 besteht aus einem konisch zulaufenden, an das Endsegment 20 angeformten, vorspringenden Ring. Der Stopper 48 begrenzt die Einführungstiefe des Darmrohrs. Dadurch werden Verletzungen bei der Anwendung sicher vermieden. Nur die drei Abschnitte 20, 22 und 24 des Spülkopf-Aufsatzes 12 können in den Darm eingeführt werden.

Beispielhaft sind die Abmessungen für den Spülkopf-Aufsatz 12 für einen Erwachsenen angegeben (die Bereichsangaben beziehen sich auf Figur 19):

| Längen | Durchmesser am Scheitelpunkt |
|---|---|
| von a bis b = 28 mm | Bereich 3: 25 mm |
| von a bis c = 100 mm | Bereich 4: 20 mm |
| von c bis d = 40 mm | Bereich 5: 16 mm |
| von d bis e = 104 mm | |
| f = 12,5 mm, Winkel ca. 60° | |

Man erkennt, dass der Stopper 48 einen ca. 50 mm Durchmesser hat und daher praktisch nicht einführbar ist. Die oben aufgeführten Abmessungen können individuell an die Bedürfnisse des Nutzers angepasst werden. Eine hyperbelförmige Wandung, die der natürlichen spastischen Verschlussreaktion der Schließmuskulatur angepasst ist, erleichtert die Einführung.

Zwischen den zusammengesetzten Abschnitten 20, 22 und 24 sind im Bereich der Verbindungsstellen Verjüngungen 49, 50 und 52 gebildet. Diese erlauben einen Verschluss während des Einführens, so dass der Nutzer sich nicht beschmutzt.

In einem nicht dargestellten Ausführungsbeispiel ist der Spülkopf-Aufsatz 12 aus einem leicht zu reinigenden und desinfizierenden Material ausgebildet. In Figur 35 und 36 wird auf den Spülkörper-Aufsatzes 12 das Schutzkondom 74 mit integriertem Gleitmittelreservoir 75 aufgesetzt. Das Schutzkondom 74 ist im vorliegenden Ausführungsbeispiel doppelwandig ausgebildet. Es ist in Figur 37 noch einmal separat dargestellt. Es besteht aus einer kräftigeren, d.h. weniger zerreißenden Rückwand und einer dünneren Vorderwand. Beim Einführen des Spülkörperaufsatzes 12 wird die dünnere Vorderwand des Schutzkondoms 74 in den Sollbruchlinien aufgerissen, so dass das Gleitmittel frei wird.

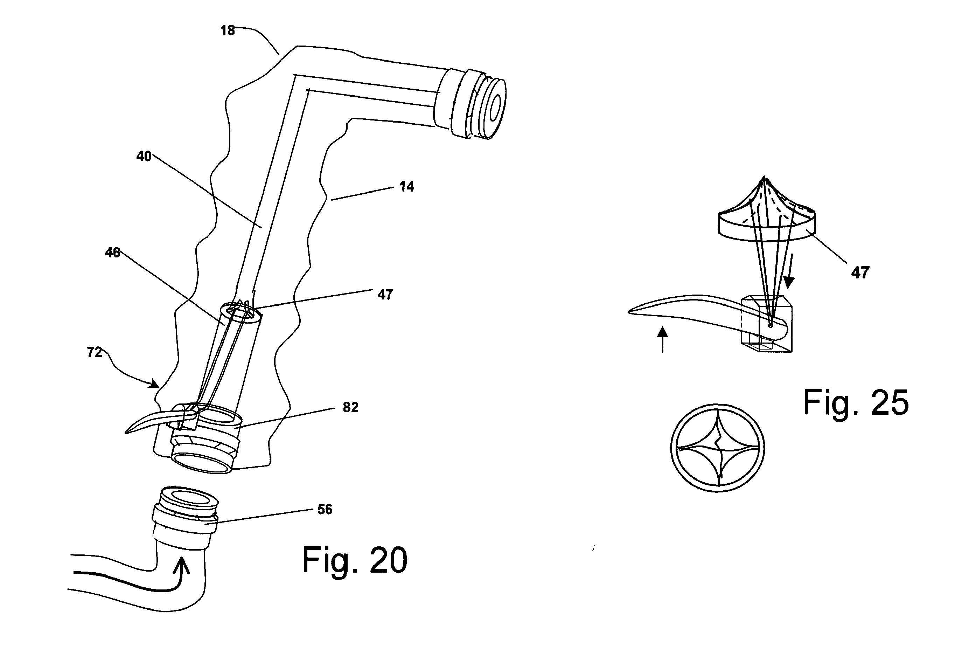

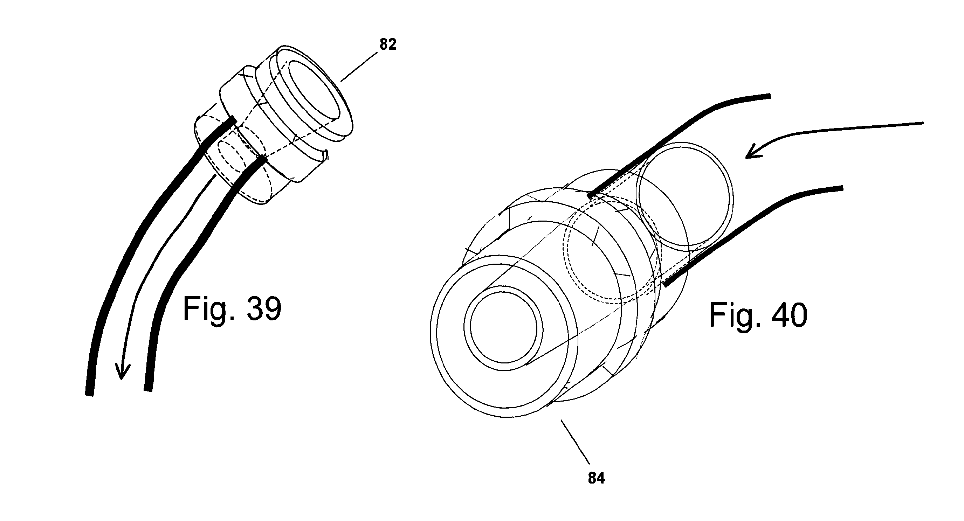

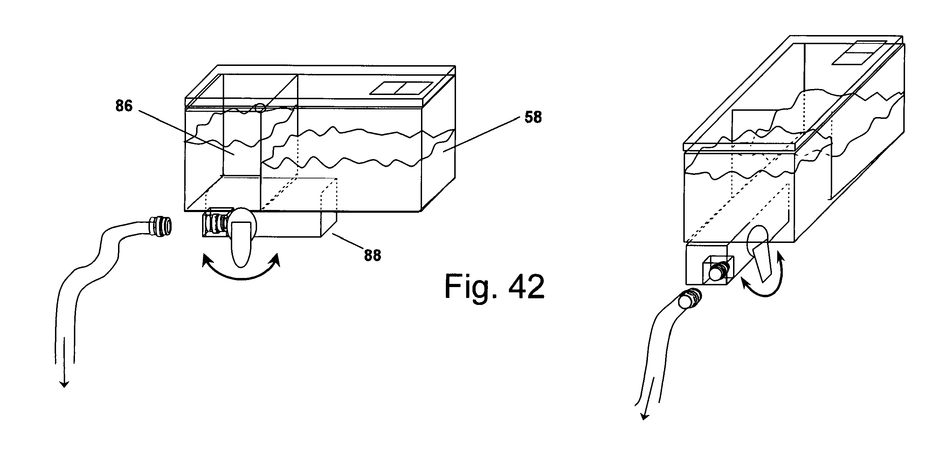

Figuren 39 bis 47 illustrieren die Flüssigkeitsversorgung des Darmrohrs 10. Das Griffteil 14 ist mit einem Muffenanschluss 82 versehen. An den Muffenanschluss kann ein in Figur 40 im Detail dargestellter Steckanschluss 84 angeschlossen werden. Der Zufuhrsilikonschlauch 56 wird mit Flüssigkeit aus einem Flüssigkeitsreservoir in Figur 42 z.B. dargestellten wärmereguliertem Boiler 86 gespeist. Im vorliegenden Ausführungsbeispiel ist das wärmeregulierende Boiler in den Spülkasten einer Toilette integriert, sämtliche anderen wärmeregulierten Flüssigkeitszuführungen sind denkbar. Die Flussmenge reguliert der Nutzer über das integrierte Steuer-/Rückschlagventil des Griffteils 14 in Figur 25 separat abgebildet.

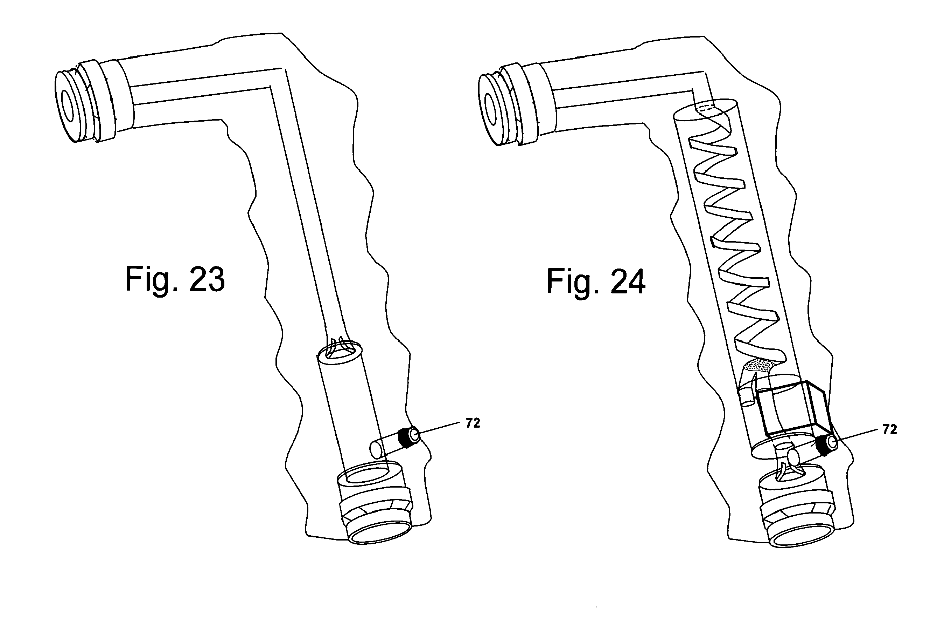

Im Bereich 46 des Griffteils 14 ist ferner ein Luer-Lock Anschluss 72 vorgesehen. Dies ist in Figur 23 dargestellt. Über diesen Anschluss 72 können der Spülflüssigkeit Reinigungsmittel, etwa Glycerin oder Elektrolyte zugeführt werden.

Ein besonderes Ausführungsbeispiel wird in Figur 21 dargestellt, bei der der Griffteil mit integriertem Schaumgenerator 16, bestehend aus einem Schaumerzeugungsrohr und einer Zuführung für das Verschäumungsmedium z.B. flotten Luftgemisch oder einer spezifischen bakteriellen Nährlösung dargestellt. Das Schaumerzeugungsrohr ist als Spirale oder Wendel ausgebildet, zur Erzielung einer größeren Turbulenz im inneren des Schaumerzeugungsrohres. Siehe hierzu Offenlegungsschrift DE 33 28 530 A1.

Das beschriebene Darmrohr 10 arbeitet wie folgt: Zunächst wird ein segmentierter Spülkopf-Aufsatz 12 auf das Griffteil 14 aufgesteckt. Das Darmrohr 10 kann nun mit dem Spülkopf-Aufsatz 12, auf den das Schutzkondom 74 mit integriertem Gleitmittelreservoir 75 aufgezogen ist, schrittweise in den Darm eingeführt werden. Der Nutzer hält das Griffteil 14 bauchseits und führt das Darmrohr mit dem darmseitigen Eingangssegment 24 schräg nach oben in die aborale Körperöffnung (Anus) ein. Das Schutzkondom 74 enthält in seinem Gleitmittelreservoir 75 eine Salbe, die neben guten Gleiteigenschaften auch ein lokal anzuwendendes Betäubungsmittel aufweist. Die äußere Hülle des Schutzkondoms zerreißt bei der Einführung und das Gleitmittel wird frei gesetzt. Das enthaltende Betäubungsmittel führt zu einer Vermeidung von Schmerzen und zur Entspannung des Schließmuskels. Wenn das Darmrohr 10 bis zum Stopper 48 eingeführt ist, wird die Flüssigkeitszufuhr, siehe hierzu als Beispiel Figur 12, mittels des Steuerventils geöffnet. Es werden vom Nutzer durch sein Gefühl bestimmt ca. 200 ml warmes Wasser eingefüllt. Dann wird das Steuerventil wieder geschlossen. Das Füllungsgefühl beginnt mit 200ml, eine Sicherheit besteht mindestens bis 500ml. Wasser fließt durch das Darmrohr 10 in den Darm. Während der Spülung kann das Darmrohr 10 leicht gedreht werden. Die Spülflüssigkeit fließt dann durch die außeraxiale Lage des Kanals 40 kreisförmig entlang der Darminnenwand.

## Patentansprüche

1. Darmrohr-Anordnung zur Applikation einer Flüssigkeit in den Dickdarm eines Lebewesens, wobei die Darmrohr-Anordnung ein Griffteil (109) und einen mit dem Griffteil (109) verbundenen Spülkörper (108) umfasst, wobei der Spülkörper (108) einen Fluidkanal (40) umfasst, **dadurch gekennzeichnet, dass**
der Spülkörper (108) mindestens zwei in den Dickdarm eines Lebewesens einführbare Segmente (201, 202, 203) umfasst, wobei jedes Segment (201, 202, 203) eine Mittelachse (118), eine Grundfläche (117, 127, 137), eine Deckfläche (116, 126, 136), einen ersten Bereich (111) und einen zweiten Bereich (112) umfasst, wobei der erste Bereich (111) zwischen der Grundfläche (117, 127, 137) und dem zweiten Bereich (112) angeordnet ist und der zweite Bereich (112) zwischen dem ersten Bereich (111) und der Deckfläche (116, 126, 136) angeordnet ist, und wobei die Länge (152) des zweiten Bereiches (112) parallel zur Mittelachse (118) größer ist als die Länge (151) des ersten Bereiches (111) parallel zur Mittelachse (118), der Durchmesser (150) des ersten Bereiches (111) in Bezug auf die Mittelachse (118) in Richtung der Deckfläche (116, 126, 136) stetig zunimmt und der Durchmesser des zweiten Bereiches (112) in Bezug auf die Mittelachse (118) in Richtung der Deckfläche (116, 126, 136) stetig abnimmt.

2. Darmrohr-Anordnung nach Anspruch 1,
**dadurch gekennzeichnet, dass**
der Spülkörper (108) ein distales Ende (110) und ein proximales Ende (120) umfasst und der maximale Durchmesser (150) der Segmente (201, 202, 203) in Bezug auf ihre jeweilige Mittelachse (118, 211, 212) vom distalen Ende (110) zum proximalen Ende (120) hin zunimmt.

3. Darmrohr-Anordnung nach Anspruch 1 oder Anspruch 2,
**dadurch gekennzeichnet, dass**
der Durchmesser (150) der Grundfläche (117, 127, 137) mindestens eines Segments (201, 202, 203) zwischen 5% und 15% geringer ist als der maximale Durchmesser (150) des Segments (201, 202, 203).

4. Darmrohr-Anordnung nach einem der Ansprüche 1 bis 3,
**dadurch gekennzeichnet, dass**
der maximale Durchmesser (150) einander benachbarter Segmente (201, 202, 203) zwischen 20% und 40% voreinander abweicht.

5. Darmrohr-Anordnung nach einem der Ansprüche 1 bis 4,
**dadurch gekennzeichnet, dass**
der Spülkörper (108) ein distales Ende (110) umfasst und die Deckfläche (116) des am distalen Ende (110) angeordneten Segments (201) konvex gekrümmt ist.

6. Darmrohr-Anordnung nach einem der Ansprüche 1 bis 5,
**dadurch gekennzeichnet, dass**
das Griffteil (109) oder der Spülkörper (108) einen nicht in den Darm einführbaren Bereich (48) mit einer Mittelachse umfasst, wobei der nicht in den Darm einführbare Bereich (48) eine Ausdehnung senkrecht zur Mittelachse aufweist, die größer ist als der maximale Durchmesser der Segmente (201, 202, 203) des Spülkörpers (108).

7. Darmrohr-Anordnung nach einem der Ansprüche 1 bis 6,
**dadurch gekennzeichnet, dass**
die Segmente (201, 202, 203) lösbar miteinander verbunden sind und/oder das Griffteil (109) und der Spülkörper (108) lösbar miteinander verbunden sind.

8. Darmrohr-Anordnung nach einem der Ansprüche 1 bis 7,
**dadurch gekennzeichnet, dass**
mindestens ein Segment (201, 202, 203) einen im Bereich der Mittelachse des Segments (201, 202, 203) angeordneten Fluidkanal (40) umfasst und/oder mindestens ein Segment (201, 202, 203) einen Fluidkanal (40) umfasst, der eine Einlassöffnung umfasst, die an der Grundfläche (117, 127, 137) des Segments (201, 202, 203) im Bereich der Mittelachse (118, 211, 212) des Segments (201, 202, 203) angeordnet ist, und eine Auslassöffnung umfasst, die die an der Deckfläche (116, 126, 136) des Segments (201, 202, 203) in radialer Richtung von der Mittelachse (118, 211, 212) des Segments (201, 202, 203) beabstandet angeordnet ist.

9. Darmrohr-Anordnung nach einem der Ansprüche 1 bis 8,
**dadurch gekennzeichnet, dass**
die Grundfläche (117, 127, 137) und die Deckfläche (116, 126, 136) mindestens eines Segments (201, 202, 203) jeweils eine Flächennormale (156, 157) aufweist, wobei die Flächennormale (157) der Grundfläche (117, 127, 137) mit der Flächennormale (156) der Deckfläche (116, 126, 136) einen Winkel (β, γ) zwischen 0° und 80° einschließt.

10. Darmrohr-Anordnung nach einem der Ansprüche 1 bis 9,
**dadurch gekennzeichnet, dass**
der Spülkörper (108) mindestens einen Verjüngungsbereich (250, 260) umfasst, der zwischen zwei Segmenten (201, 202, 203) angeordnet ist und der eine Grundfläche (257, 267) mit einer Flächennormale (259, 269) und eine Deckfläche (256, 266) mit einer Flächennormale (258, 268) umfasst, wobei die Flächennormale (259, 269) der Grundfläche (257, 267) mit der Flächennormale (258, 268) der Deckfläche (256, 266) einen Winkel (β, γ) zwischen 0° und 80° einschließt.

11. Darmrohr-Anordnung nach einem der Ansprüche 1 bis 10,
**dadurch gekennzeichnet, dass**
das Griffteil (109) eine Mittelachse (119) umfasst, der Spülkörper (108) ein proximales Ende (120) und eine Mittelachse (118) im Bereich des proximalen Endes (120) umfasst und die Mittelachse (119) des Griffteils (109) mit der Mittelachse (118) des Spülkörpers (108) im Bereich des proximalen Endes (120) einen Winkel α zwischen 50° und 90° einschließt.

12. Darmrohr-Anordnung nach einem der Ansprüche 1 bis 11,
**dadurch gekennzeichnet, dass**
das Griffteil (109) und/oder der Spülkörper (108) eine Vorrichtung zum Einbringen einer Flüssigkeit in den Fluidkanal (40) des Spülkörpers (108) umfasst und/oder mit einer Vorrichtung zum Einbringen einer Flüssigkeit in den Fluidkanal (strömungstechnisch) verbunden ist.

13. Darmrohr-Anordnung nach einem der Ansprüche 1 bis 12,
**dadurch gekennzeichnet, dass**
der Spülkörper (108) einen Aufsatz (101) umfasst, welcher die äußere Oberfläche mindestens eines Segmentes (201, 202, 203) zumindest teilweise bedeckt.

14. Darmrohr-Anordnung nach Anspruch 13,
**dadurch gekennzeichnet, dass**
der Aufsatz (21, 101) als Schutzkondom (101) ausgestaltet ist, welches die äußere Oberfläche mindestens eines Segmentes (201, 202, 203) zumindest teilweise bedeckt.

15. Darmrohr-Anordnung nach Anspruch 13 oder Anspruch 14,
**dadurch gekennzeichnet, dass**
der Aufsatz (21, 101) ein Reservoir umfasst.

16. Griffteil (109) für eine Darmrohr-Anordnung zur Applikation einer Flüssigkeit in den Dickdarm eines Lebewesens gemäß einem der Ansprüche 1 bis 15,
**dadurch gekennzeichnet, dass**
das Griffteil (109) eine Vorrichtung zur Steuerung der Zufuhr der zu applizierenden Flüssigkeit in einen Fluidkanal (40) der Darmrohr-Anordnung umfasst.

17. Spülkörper (108) für eine Darmrohr-Anordnung zur Applikation einer Flüssigkeit in den Dickdarm eines Lebewesens, (gemäß einem der Ansprüche 1 bis 15,) wobei der Spülkörper einen Fluidkanal (40) umfasst,
**dadurch gekennzeichnet, dass**
der Spülkörper (108) mindestens zwei in den Dickdarm eines Lebewesens einführbare Segmente (201, 202, 203) umfasst, wobei jedes Segment (201, 202, 203) eine Mittelachse (118), eine Grundfläche (117, 127, 137), eine Deckfläche (116, 126, 136), einen ersten Bereich (111) und einen zweiten Bereich (112) umfasst, wobei der erste Bereich (111) zwischen der Grundfläche (117, 127, 137) und dem zweiten Bereich (112) angeordnet ist und der zweite Bereich (112) zwischen dem ersten Bereich (111) und der Deckfläche (116, 126, 136) angeordnet ist, und wobei die Länge (152) des zweiten Bereiches (112) parallel zur Mittelachse (118) größer ist als die Länge (151) des ersten Bereiches (111) parallel zur Mittelachse (118), der Durchmesser (150) des ersten Bereiches (111) in Bezug auf die Mittelachse (118) in Richtung der Deckfläche (116, 126, 136) stetig zunimmt und der Durchmesser des zweiten Bereiches (112) in Bezug auf die Mittelachse (118) in Richtung der Deckfläche (116, 126, 136) stetig abnimmt.
